# EUROPEAN PATENT APPLICATION

(11) **EP 3 971 575 A1**
(43) Date of publication of application: **23.03.2022**
(21) Application number: 20196899.7
(22) Date of filing: 18.09.2020
(51) Int. Cl.: G01N 33/84, G01N 33/50, G01N 33/58

(54) **FLUORESCENT PROBES FOR QUANTIFICATION OF FREE COPPER**

(71) Applicant: Technische Universität Berlin, 10623 Berlin (DE)
(72) Inventor: Dr. Yücesan, Gündog, 13347 Berlin (DE); Prof. Dr. Haase, Hajo, 10245 Berlin (DE)
(74) Representative: Hertin und Partner Rechts- und Patentanwälte PartG mbB

(57) **Abstract**

The present invention relates to a method for detecting free metal ions in a sample comprising, providing a liquid sample potentially comprising free metal ions, adding to said sample a fluorescent probe comprising an organic fluorescent core and one or more metal binding functional group, wherein the one or more metal binding functional group is selected from the group comprising a phosphonic acid group and an arsonic acid group and is covalently linked to a sp or a sp²-carbon atom or a nitrogen atom of the fluorescent core via a P or As atom, and measuring fluorescence of said sample. In embodiments, presence of the metal ions leads to a concentration-dependent decrease, increase or shift of fluorescence compared to a reference sample, wherein the method preferably involves determining a concentration of metal ions in said sample. In embodiments, the liquid sample is a biological sample, such as a bodily fluid, a tissue sample or a sample comprising cells.

## Description

### DESCRIPTION

The present invention relates to a method for detecting free metal ions in a sample comprising, providing a liquid sample potentially comprising free metal ions, adding to said sample a fluorescent probe comprising an organic fluorescent core and one or more metal binding functional group, wherein the one or more metal binding functional group is selected from the group comprising a phosphonic acid group and an arsonic acid group and is covalently linked to a sp or a sp²-carbon atom or a nitrogen atom of the fluorescent core via a P or As atom, and measuring fluorescence of said sample. In embodiments, presence of the metal ions leads to a concentration-dependent decrease, increase or shift of fluorescence compared to a reference sample, wherein the method preferably involves determining a concentration of metal ions in said sample. In embodiments, the liquid sample is a biological sample, such as a bodily fluid, a tissue sample or a sample comprising cells.

### BACKGROUND OF THE INVENTION

Nearly half of all cellular proteins require one or more metal ions as cofactors (transition metals to alkali and alkaline-earth metals) to perform their functions¹. Metal ions are present in all 6 classes of enzymes² and metalloproteins assume significant roles in signal transduction³⁻⁶. Therefore, the distribution of metal ions in biology is very tightly regulated through a complex network of interactions ensuring proper metal ion homeostasis ^{7,8}. Disturbance or mutations in the metabolic pathways of metal ion homeostasis could produce significant disarray in signal transduction or impede other biochemical pathways ^{3-6,9}, resulting in cellular damage or even death, e.g., by apoptosis ¹⁰⁻¹², and promote diseases such as cancer ¹³, diabetes ¹⁴⁻¹⁶, vascular ¹⁷ or soft tissue calcifications ¹⁸ and Alzheimer's disease ¹⁹⁻²¹. Therefore, monitoring the concentrations of metal ions in living systems is crucial to provide diagnosis and treatment of innumerous metabolic disorders as well as to understand the mechanism of how metal ions are regulated ²²⁻²⁴.

Despite the importance of metal ions in biology, there are still only a few methods to monitor free or unbound metal ion concentrations; among them, fluorescent imaging is one of the most suitable techniques ²⁵⁻²⁷. Small molecule fluorescent sensors could generate response upon metal binding ^{24,28-31} or protein and peptide-based systems mobilize fluorescent compartments to produce FRET fluorescence upon metal binding ³²⁻³⁵. However, the number of such fluorescent probes is limited, and design routes to create metal ion responsive fluorescence are not yet well-established.

Accordingly, there is a need in the art to provide a class of fluorescent probes that enable determining the concentrations of metal ions in a sample, wherein the probes can be easily synthetized by routine procedures and the probe design can be adjusted to specific needs, such as detection of specific metal ions in a sample.

The ideal fluorescent probe to detect metal ions should provide dynamic electronic interactions between the fluorescent core and the metal ion to cause a detectable change in fluorescence. To date, no probes optimally suited for these applications are available, which would be highly valuable for reliably measuring metal ion concentrations in biological samples.

### SUMMARY OF THE INVENTION

In light of the prior art the technical problem underlying the present invention is the provision of fluorescent probes and a method using such probes for detecting free metal ions in a sample, such as a biological sample, wherein the probes provide dynamic electronic interactions between a fluorescent core of the probe and the bound metal ion to cause a detectable change in fluorescence.

This problem is solved by the features of the independent claims. Preferred embodiments of the present invention are provided by the dependent claims.

The invention therefore relates to a method for detecting free metal ions in a sample comprising,
- providing a liquid sample potentially comprising free metal ions,
- adding to said sample a fluorescent probe comprising
   i. an organic fluorescent core and
   ii. one or more metal binding functional group,
   iii. wherein the one or more metal binding functional group is selected from the group comprising a phosphonic acid group and an arsonic acid group and is covalently linked to a sp or a sp²-carbon atom or a nitrogen atom of the fluorescent core via a P or As atom,
- and measuring fluorescence of said sample.

The present invention is based on the unexpected idea that a metal sensing unit (metal binding functional group) of the probe of the invention should be connected to the organic fluorescent core directly via at least one of the sp or sp²-carbon atoms and/or via at least one nitrogen atom to produce extended conjugation interacting with the target metal. In embodiments, the metal binding functional group being selected from the group comprising or consisting of a phosphonic acid group and an arsonic acid group are covalently linked to a sp or a sp²-carbon atom or a sp or a sp²-nitrogen atom of the fluorescent core via a P or As atom.

In the context of the invention, it is understood that an organic fluorescent core is a fluorescent organic molecule/structure comprising one or more (combined) aromatic groups, and/or planar or cyclic structures with several π bonds, which form a conjugated system of delocalized electrons.

As a consequence, interaction of a probe of the invention with a metal ion via the metal binding group affects the conjugated system of electrons resulting in a modification of the fluorescent properties of the fluorescent core, since the conjugated system extends to the metal binding group.

In contrast, sp³ bonded metal sensing units that are connected to a fluorescent core can mediate binding of such a probe to a metal ion, but binding of the metal ion does not influence the fluorescent properties of the probe, at least not to the same extend as it is the case for a probe of the method presented herein, since the conjugated electron system is not in contact with the metal binding group and the binding metal ion.

In embodiments of the invention, the metal binding group is connected to the fluorescent core via phenylphosphonic acid tethers. Phenylphosphonic acid tethers provide 1.7 and 7.4 pKa1 and pKa2 values, respectively, and each of the phenylphosphonic acid tethers are expected to provide -2 negative charge at physiological pH.

Therefore, phosphonic acid derivatives are suitable to generate ionic interactions with divalent metal ions in biological systems, but their use as metal sensing units has been neglected due to difficult and limited synthetic routes, especially due to the challenge of forming P-C bonds in conjugated fluorescent systems.

In embodiments of the invention, the one or more metal binding functional group is selected from the group comprising a phosphonic acid group and an arsonic acid group and is covalently linked to a sp²-carbon atom or a nitrogen atom of the fluorescent core via a P or As atom.

In embodiments, the probes comprise conjugated/aromatic tethers, which can be regarded as an extension of the conjugated system of the fluorescent core to the location of the metal binding functional group, it is decisive that the metal binding groups are connected to the fluorescent core via a continuous system of delocalized electrons for enabling detectable modification of the fluorescent properties by metal ion binding. This extension of the conjugated system is brought about by binding the P or As atom of the metal binding phosphonic acid or arsonic acid group directly to an sp or sp² C or N atom of the fluorescent core, or by connecting the metal binding functional group and the sp or sp² C or N atom of the functional groups with tethers that expand the delocalized electron system of the fluorescent core to the functional metal binding group.

In embodiments, the method of the invention is performed at a pH in the range of 6-9, preferably at a pH of about 7-8, such as about 7.4. pH conditions in the range of a physiological pH are preferred since the method is preferably performed on biological samples, such as samples comprising cells or body fluids.

In embodiments of the method of the invention the organic fluorescent core is selected from the group comprising tetrapyrrole derivatives, such as porphyrin or phthalocyanine, acridine, BODIPY, cyanine or cyanine derivatives, carbazole, coumarin or coumarin derivatives, xanthene or xanthene derivatives such as fluorescein or rhodamine.

It surprisingly turned out that the concept of connecting the metal binding functional group directly to a sp² or sp carbon or nitrogen atom that forms part of the delocalized electron system of the fluorescent core is applicable to various kinds of fluorescent cores.

In embodiments of the method of the invention, the fluorescent probe comprises two or more metal binding functional groups.

It was surprisingly found out that the inclusion of two metal binding groups as compared to only one is advantageous, since upon binding of two metal ions, such as one metal ion to each of the groups, the detectable change of fluorescence increases in comparison to a corresponding probe with only one metal binding group. This holds also true for embodiments where one metal ion can bind to two metal binding groups of a probe. Accordingly, the use of probes with more metal binding groups is advantageous. Therefore, in preferred embodiments, the probes comprise 2, 3, 4, 5, 6, 7, 8 or more metal binding functional groups, such as phosphonic acid groups and/or arsonic acid groups. In embodiments, such an increase in the number of metal binding phosphonic acid or arsonic acid functional groups improves the sensitivity and allows fine-tuning of the affinity.

In embodiments of the invention, one metal ion can bind to more than one metal binding groups, such as to two metal binding groups, wherein the different metal binding groups bound by one metal ion can be comprised in the same probe molecule or can be of different probe molecules.

The method according to any one the preceding claims, wherein the fluorescent probe is selected from the group comprising
- 5,10,15,20-tetrakis[*p*-phenylphosphonic acid] porphyrin (*p*-H₈TPPA), and
- 5,10,15,20-tetrakis[*m*-phenylphosphonic acid] porphyrin (*m*-H₈TPPA).

As shown in the examples below, *p*-H8TPPA and *m*-H8TPPA change their fluorescent properties upon binding of various metal ions, wherein the changes appear to be metal ion specific. Accordingly, the probes are suitable for detection of various metal ions as disclosed herein.

In embodiments, measuring fluorescence is performed by exciting the sample with light of an excitation wavelength and detecting emitted light at an emission wavelength. Accordingly, it is possible to detect the presence and changes of metal ion presence and preferably concentration by performing standard fluorescence measurements on a sample comprising the probe of the invention.

Certain embodiments comprise the investigation of one or more metal ions in biological fluids such as plasma, serum, urine, saliva, cerebrospinal fluid, cell or tissue lysates, as well as measurements in cell culture, isolated organs (ex vivo) or in vivo. Also, other fluids can be investigated, e.g., liquids intended for human consumption, in particular food stuffs such as beverages. All previously mentioned liquids could either be measured in concentrated form (by direct addition of the probe) or after dilution in a suitable solvent or buffer. In the case of solid samples, these might be liquified or dissolved prior to analysis. The invention also comprises the use of the probes in the form of point-of-care devices, such as the probe being present in a dried form on paper or a comparable carrier material, intended to be brought into contact with a liquid sample for analytical purposes.

In embodiments, presence of the metal ions leads to a concentration-dependent decrease, increase or shift of fluorescence compared to a reference sample, wherein the method preferably involves determining a concentration of metal ions in said sample.

Preferably, the metal ions are ions of a group of metals comprising Cu, Zn, Pb, Hg, Cd, Co, Mg, Ca and Mn, wherein the ions are preferably divalent.

In certain embodiments, the metal ions are ions of a group of metals comprising Cu, Zn, Pb, Hg, Cd, Co, and Mn, wherein the ions are preferably divalent. It surprisingly turned out that in presence of free divalent ions of these metals the fluorescence emitted by the probes as described herein, in particular *p*-H₈TPPA and *m*-H₈TPPA, decreased remarkably, enabling to detect the presence of such metal ions by detecting a decrease in fluorescence.

Importantly, it was possible to show that the detected changes in fluorescence of the probes brought about by binding of the metal ions were concentration dependent. Consequently, in embodiments, the method of the invention can also be used for determining the concentration of specific metal ions in a sample.

In preferred embodiments, the metal ions are copper ions, preferably Cu²⁺, and the fluorescent probe is *p*-H₈TPPA or *m*-H₈TPPA.

In embodiments, the concentration of Cu²⁺, Zn²⁺, Co²⁺, Mn²⁺, Pb²⁺, Hg²⁺ or Cd²⁺ can be determined due to concentration dependent decrease of fluorescence emitted by a probe of the invention, in particular *p*-H₈TPPA or *m*-H₈TPPA.

The presence and concentration of copper ions, in particular Cu²⁺ ions, in a sample can be determined by the method of the invention, wherein preferably *p*-H₈TPPA or *m*-H₈TPPA are used as fluorescent probes. In the context of such a method, the presence of Cu²⁺ ions leads to a concentration dependent decrease of fluorescence emitted by the probe, preferably *p*-H₈TPPA or *m*-H₈TPPA.

In embodiments, the metal ions are ions of a group of metals comprising Fe, Mg and Ca, wherein the ions are preferably divalent. It was surprisingly discovered that divalent cations of Fe, Mg and Ca induce a fluorescent increase in the probes of the present invention, in particular when *p-*H₈TPPA or *m*-H₈TPPA were used.

Accordingly, in embodiments presence of the metal ions, such as divalent cations of Fe, Mg and Ca, leads to a concentration dependent increase of fluorescence compared to a reference sample. In embodiments, the method of the invention involves determining a concentration of metal ions in a sample.

In embodiments, it is possible to differentially determine the presence and concentration of various metal ions present in a sample, since binding of different ions has differential effects on the fluorescence of the probe.

In embodiments, the method of the invention comprises a step of adding a metal chelator to the sample. By means of a chelator that can be specific for a certain metal ion or for a certain group of metal ions, the method of the invention can be used to determine the presence and/or concentration of a specific metal ion or group of metal ions that are not affected by the chelator, while metal ions that are sequestered by the chelator cannot interfere with the fluorescent signal generated by the probe.

In embodiments relating to complex samples containing several different metal ions, selective chelating agents may be used for enrichment of the analyte (metal ion(s) of interest) or removal of metal ions potentially interfering with the signal of the chosen analyte. In embodiments with low analyte concentration in a sample, detection limits may be improved by pre-concentration, e.g., precipitation, dialysis, size-exclusion centrifugation, and/or freeze drying and/or other methods for evaporation of solvents.

In preferred embodiments of the invention, the liquid sample is a biological sample, such as a bodily fluid, a tissue sample or a sample comprising cells. Accordingly, the method of the invention can be used to determine the presence and preferably concentration of metal ions in a bodily sample isolated from a patient, or in a biological system, such as a cell culture system.

Preferably, the sample comprises cells and the fluorescent probe is cell permeable. Such embodiments enable determining the presence of specific metal ions, such as Cu²⁺, in a cell, for example by measuring fluorescence by fluorescence microscopy, flow cytometry or fluorimeters.

In embodiments, the fluorescence measurement is performed in association with a microscopic analysis of the cells, such as confocal fluorescent microscopy. Confocal microscopy enables quantification of fluorescence in association with the location of the fluorescent signal within the cell. Accordingly, it is possible to localize areas of high and low metal iron concentration within the same cell.

In embodiments, the concentration of free metals is calculated based on the mass action law, in particular involving calibration based on addition of a chelator and/or metal ions.

The use of chelators can be particularly advantageous in the context of the present invention, since it was surprisingly shown, that the effect of binding of specific metal ions to the probes of the invention, such as *p*-H₈TPPA or *m*-H₈TPPA can be reversed by addition of a chelator to the sample, while for other metal ions such modifications of fluorescence were not reversible.

Accordingly, in embodiments of the invention the method may involve parallel measurements of the same sample, which has been split in two, wherein in case of a first of the resulting two samples a chelator has been added before adding the probe, and in the second of the resulting sample no chelator has been added.

Furthermore, chelators could be added to a sample after measuring fluorescence in the absence of the chelator, to determine reversibility of the fluorescence modification of the probe by sequestration of free metal ions.

The use of chelators enables various possibilities of modifying the method of the invention, since it was surprisingly found that for example the fluorescent quenching observed by Zn²⁺ and Hg²⁺ could be reversed by subsequent addition of EDTA or EGTA, while a Cu²⁺ mediated quenching was almost not affected by the chelators. Based on the data presented herein, the person skilled in the art can design specific methods that are directed to the detection of specific metal ions, also in complex samples comprising various metal ions, since it is shown herein that the probes of the invention react differentially to the presence of different metal ions.

In embodiments, the method of the invention is used in clinical diagnostics for determining available levels of free metal ions in a patient sample. Such diagnostic methods can be used for identifying medical conditions that are associated with deviations of metal ion concentrations from the standard range in healthy individuals.

Embodiments of the invention relating to the use of the inventive method in the context of a diagnostic method comprise diagnostic methods directed to detection of characteristic changes in metal ion homeostasis that are typically associated with certain diseases, e.g., changes in the homeostasis of Zn, Fe, Cu during infection and inflammation or during malignancies. Other applications for diagnostic purposes included changes resulting from inadequate nutrition, which could be detected by measuring the respective micronutrient concentration in patient samples, typically plasma, serum or urine.

Furthermore, the invention relates to a method for diagnosis, prognosis, risk assessment, monitoring, therapy guidance and/or therapy control of a medical/clinical condition of a subject associated with changes in the level of metal ions in a bodily fluid or bodily sample, the method comprising performing the method for detecting free metal ions in a sample as described herein, wherein the sample has been isolated from a patient or comprises biological material that has been isolated from a patient.

In embodiments, the method of the invention is used as a procedure in clinical diagnostics for determining available levels of free metal ions as a diagnostic marker for nutrient supply.

In embodiments, the methods described herein are in vitro methods.

### DETAILED DESCRIPTION OF THE INVENTION

All cited documents of the patent and non-patent literature are hereby incorporated by reference in their entirety.

The present invention is directed to a method for detecting free metal ions in a sample comprising, providing a liquid sample potentially comprising free metal ions; adding to said sample a fluorescent probe comprising an organic fluorescent core and one or more metal binding functional group; wherein the one or more metal binding functional group is selected from the group comprising a phosphonic acid group and an arsonic acid group and is covalently linked to a sp or a sp²-carbon atom or a nitrogen atom of the fluorescent core via a P or As atom.

As used herein, the term "free metal ion" refers to any kind of metal ion that is dissolved in a liquid, such as water, and which is not tightly bound by other molecular structures (either macromolecules (e.g., proteins) or low molecular weight ligands, solid salts or the like, but is readily exchangeable and therefore available for binding to the fluorescent probe.

Metal ions that can be detected using the method of the invention comprise any kind of metal cation that is formed by metal atoms. Metals in the sense of the invention comprise all metals known to a person skilled in the art. This includes, without limitation, cations of alkali metals, including lithium (Li), sodium (Na), potassium (K), rubidium (Rb), cesium (Cs), and francium (Fr), as well as of alkaline earth metals, including beryllium (Be), magnesium (Mg), calcium (Ca), strontium (St), barium (Ba) and radium (Ra).

Particularly preferred metal ions of the invention are metal ions with relevance in biology and in biological systems. Metal ions have been associated with biological systems for billions of years, and major (iron, manganese, magnesium and zinc) and minor (copper, cobalt, nickel, molybdenum, tungsten) metal ions have become aligned with living organisms through the interplay of biogeochemical weathering and metabolic pathways involving the products of that weathering. Organisms require redox reactions to induce metabolism and other life processes, and metals have a tendency to lose electrons and are important for redox reactions. Metals have become so central to cellular function that the collection of metal-binding proteins (referred to as the metallomes) accounts for over 30% of all proteins in the cell. Metals are known to be involved in over 40% of enzymatic reactions, and metal-binding proteins carry out at least one step in almost all biological pathways. Metals are also toxic so a balance must be acquired to regulate where the metals are in an organism as well as in what quantities. Metals and metal ions with relevance in biological systems are naturally occurring elements that have a tendency to undergo oxidation. Vanadium, molybdenum, cobalt, copper, chromium, iron, manganese, nickel, and zinc are deemed essential because without them biological function is impaired. Biologically relevant metal ions of the invention comprise ions of magnesium (Mg), manganese (Mn), iron (Fe), nickel (Ni), cobalt (Co), copper (Cu), zinc (Zn), molybdenum (Mo), calcium (Ca), and tungsten (W).

Further biologically relevant metal ions are ions of metals that can be toxic, such as lead (Pb), mercury (Hg), cobalt (Co) and cadmium (Cd).

Preferably, a metal ion to be detected by the method of the invention is stable, i.e. it is not subject to a redox reaction transforming it into its atomic state and preferably do not include instable elements/metals with a low half-life time.

Preferred free metal ions that can be detected and preferably quantified by the method of the invention comprise Cu, Zn, Pb, Hg, Cd, Co and Mn, preferably as divalent ions. It was surprisingly found that binding of free divalent ions of these metals leads to a fluorescent decrease of *p-*H₈TPPA or *m*-H₈TPPA.

Further metal ions of the invention comprise Fe, Mg, and Ca, preferably in their divalent form. It was surprisingly found that binding of free divalent ions of these metals leads to a fluorescent increase of *p*-H₈TPPA or *m*-H₈TPPA.

Preferably, the metal ions to be detected by the method of the invention are divalent. The valence or valency of an element is a measure of its combining power with other atoms when it forms chemical compounds or molecules, and is defined as the number of hydrogen atoms that can combine with an element in a binary hydride or twice the number of oxygen atoms combining with an element in its oxide or oxides.

A method of detecting free metal ions is a method that is used for determining the presence of free metal ions in a sample. The method can be directed to detecting a specific individual kind of metal ion, or to detection of the presence of one or more metal ions. The method can in embodiments be adapted to detect the presence of only a specific kind of metal ion in a complex mixture that may comprise multiple kinds of metal ions. In embodiments, the method is directed to determining the presence of one or more metal ions in a sample, for example a sample comprising multiple metal ions. In embodiments, the method can detect one or more of a defined group of metal ions. Depending on the specific probe and on the reference sample, it is possible to adjust the method of the present invention to specifically detect specific kinds of metal ions in a sample.

The method of detecting free metal ions can be used as a binary method, indicating either the presence or the absence of metal ions, such as a the presence or absence of one or more of a group of metal ions, or the presence or absence of a specific kind of metal ion.

In embodiments, the method of detecting free metal ions is a method of determining a concentration of metal ions in a sample. In the context of such a concentration measurement, the sample volume can be known, and the concentration can be calculated dividing the determined amount of metal ions by the known sample volume. Alternatively, the method involves the comparison of a measured fluorescence of the sample with the fluorescence measured in one or more reference samples. Such reference samples can comprise samples that do not contain any metal ions but only consist of the sample buffer used for the measurements. Further reference samples can comprise samples with known concentration of the respective metal ions to be determined. Such reference samples with known concentrations can be prepared by adding defined amounts of the respective metal ions to the sample buffer comprising the probe and determining the fluorescence after adding the fluorescent probe. By measuring multiple reference samples of different concentrations of metal ions of interest, one can establish a so-called reference standard curve which can be referred to for determining the concentration of metal ions in the sample of interest. The standard curve may also enable generation of a reference formula that can be used to calculate a metal ion concentration of a sample based on the determining fluorescence.

In embodiments comprising complex samples containing several different metal ions, selective chelating agents may be used for enrichment of the analyte/metal ion(s) of interest and/or removal of metal ions potentially interfering with the signal of the chosen analyte. For example, in case of low analyte concentration in a sample, detection limits may be improved by pre-concentration, e.g., precipitation, dialysis, size-exclusion centrifugation, and freeze drying or other methods for evaporation of solvents.

In the context of the invention, the term "liquid sample" describes any kind of liquid sample, such as a bodily fluid of an organism or an environmental water sample. Furthermore, the term liquid sample also relates to non-liquid material that can be dissolved or diluted in a liquid, such as a buffer, in order to dissolve the free metal ions comprised in sample. Such material can comprise soil samples or tissue sample, that can be added to a liquid buffer and may be subsequently subjected to a homogenization or mixing process, in order to expose the material to the buffer and to dissolve the free metal ions comprised by the material in the buffer.

In embodiments, the sample may be any kind of liquid or liquefiable material suspected to comprise free metal ions. As described above, a liquefiable material is a material that can be dissolved or diluted in a liquid, such as a buffer, in order to dissolve the free metal ions comprised in sample.

In preferred embodiments, the liquid sample is a biological sample, such as a bodily fluid, a tissue sample or a sample comprising cells. As used herein, it is understood that a biological sample is a sample comprising biological material, such as a bodily fluid, a biological tissue or biological cells.

Examples of bodily fluids comprise blood, serum, plasma, cerebrospinal fluid, urine, saliva, sputum, pleural effusions, cells, a cellular extract, a tissue sample, a tissue biopsy, a stool sample and the like. In particular, the term "liquid sample" also comprise cells and tissue cultures comprising biological cells, which can be analyzed in the context of the method of the invention.

Biological samples that can be used in the method of the invention comprise biological fluids such as plasma, serum, urine, saliva, cerebrospinal fluid, cell or tissue lysates, as well as measurements in cell culture, isolated organs (ex vivo) or in vivo. Also, other fluids can be investigated, e.g., liquids intended for human consumption, in particular food stuffs such as beverages.

As used herein, it is understood that the sample can either be an undiluted isolated liquid or a liquid or liquified sample that is diluted in a suitable buffer. Also, in the context of the method of the invention it is understood that embodiments where the probe is diluted in a buffer (or solvent) and successively added the sample, either diluted in a buffer or undiluted, are also comprised.

It is preferred that the probe to be used in the context of the present invention is cell permeable, so it can traverse the cell membrane and bind to metal ions present inside a biological cell, for example in the cytoplasm.

Cell permeable probes are probes that efficiently cross the cell membrane and access the cytosol of a cell. Molecules that can readily cross cell membranes are frequently needed in biological research and medicine and besides fluorescent probes functioning as metal ion indicators, as the probes of the present invention, cell permeability is also important for pH indicators, fluorescent dyes, crosslinking molecules, fluorogenic enzyme substrates, and various protein inhibitors that may be functioning as pharmacological drugs. Due to the extensive research in this field, it is known to a skilled person how to design or modify the probes of the invention in order to achieve cell permeability. For example, it is known to the skilled person that amphipathic molecules are likely to be cell permeable. In embodiments, charged groups can be chemically masked for enabling cell permeability, followed by enzymatic removal of the masking group (which can be, for example, an acetoxymethyl ester or an ethyl ester) once the probe is inside the cell.

A cell comprised by a liquid sample of the invention that is exposed to a cell permeable fluorescent probe as described herein may be analyzed by microscopic analysis, for example by using a confocal fluorescent microscope, in order to measure fluorescence.

In fluorescence microscopy, fluorescence is used to study the properties of organic or inorganic substances, and in particular of biological cells. A fluorescence microscope is a microscope that uses fluorescence to generate an image, whether it is a more simple set up like an epifluorescence microscope or a more complicated design such as a confocal microscope, which uses optical sectioning to get better resolution of the fluorescence image.

The basic principle of fluorescent microscopy is that a specimen is illuminated with light of a specific wavelength (or wavelengths), which is absorbed by the fluorophores comprised by the specimen (or sample), causing them to emit light of longer wavelengths (i.e., of a different color than the absorbed light). The illumination light is separated from the much weaker emitted fluorescence using a spectral emission filter. Typical components of a fluorescence microscope are a light source (xenon arc lamp or mercury-vapor lamp are common; more advanced forms are high-power LEDs and lasers), the excitation filter, the dichroic mirror (or dichroic beam splitter), and the emission filter (see figure below). The filters and the dichroic beam splitter are chosen to match the spectral excitation and emission characteristics of the fluorophore used to label the specimen. In this manner, the distribution of a single fluorophore (color) is imaged at a time. Multi-color images of several types of fluorophores must be composed by combining several single-color images.

In preferred embodiments of the invention, cells comprised by a liquid sample are analyzed by confocal microscopy. Confocal microscopy is most frequently performed as confocal laser scanning microscopy (CLSM) or laser confocal scanning microscopy (LCSM) and is an optical imaging technique for increasing optical resolution and contrast of a micrograph or a microscopy sample by means of using a spatial pinhole to block out-of-focus light in image formation. The analyzed sample may comprise a fixed or a living cell or tissue sample. Capturing multiple two-dimensional images at different depths in a sample enables the reconstruction of three-dimensional structures (a process known as optical sectioning) within an object. Confocal microscopy enables easy quantification of the acquired fluorescence data.

Light travels through the sample under a conventional fluorescent microscope as far into the specimen as it can penetrate, while a confocal microscope only focuses a smaller beam of light at one narrow depth level at a time. The CLSM achieves a controlled and highly limited depth of focus.

In the context of the invention, the term "fluorescent probe" refers to a fluorescent molecule (also called fluorophore) whose fluorescence is affected by environmental aspects such as polarity or ions, in case of the present invention by binding of metal ions to the metal binding functional group. A fluorophore is a fluorescent chemical compound that can re-emit light upon light excitation. Fluorophores typically contain several combined aromatic groups, or planar or cyclic molecules with several π bonds. Fluorophores can be used alone, as a tracer in fluids, as a dye for staining of certain structures, as a substrate of enzymes, or as a probe or indicator, when its fluorescence is affected by environmental aspects, such as binding of a ligand to the fluorophore. Importantly, fluorophores and fluorescent probes can be used to stain tissues, cells, or materials in a variety of analytical methods, i.e., fluorescent imaging, microscopy and spectroscopy.

Fluorescence is the emission of light by a substance that has absorbed light or other electromagnetic radiation. It is a form of luminescence. In most cases, the emitted light has a longer wavelength, and therefore lower energy, than the absorbed radiation. The most striking example of fluorescence occurs when the absorbed radiation is in the ultraviolet region of the spectrum, and thus invisible to the human eye, while the emitted light is in the visible region, which gives the fluorescent substance a distinct color that can be seen only when exposed to UV light. Fluorescent materials cease to glow nearly immediately when the radiation source stops, unlike phosphorescent materials, which continue to emit light for some time after.

Fluorescence is brought about by absorption of photons in the singlet ground state promoted to a singlet excited state. The spin of the electron is still paired with the ground state electron, unlike phosphorescence. As the excited molecule returns to ground state, it involves the emission of a photon of lower energy, which corresponds to a longer wavelength, than the absorbed photon.

The fluorescent probes that are used in the context of the present invention comprise an organic fluorescent core and one or more metal binding functional groups.

As used herein, the term organic fluorescent core refers to a fluorescent organic molecular structure comprising delocalized electronic structure. Delocalized electrons are electrons in a molecule that are not associated with a single atom or a covalent bond. In organic chemistry, the term delocalization refers to resonance in conjugated systems and aromatic compounds.

A conjugated system is a system of connected p orbitals with delocalized electrons in a molecule, which in general lowers the overall energy of the molecule and increases stability. It is conventionally represented as having alternating single and multiple bonds. Lone pairs, radicals or carbenium ions may be part of the system, which may be cyclic, acyclic, linear or mixed. Conjugation is the overlap of one p orbital with another across an intervening σ bond. A conjugated system has a region of overlapping p orbitals, bridging the interjacent locations that simple diagrams illustrate as not having a π bond. They allow a delocalization of π electrons across all the adjacent aligned p orbitals. The π electrons do not belong to a single bond or atom, but rather to a group of atoms.

Aromatic structures are cyclic (ring-shaped) and planar (flat) with a ring of resonance bonds that gives increased stability compared to other geometric or connective arrangements with the same set of atoms. Aromatic molecules are very stable, and do not break apart easily to react with other substances. Organic compounds that are not aromatic are classified as aliphatic compounds-they might be cyclic, but only aromatic rings have special stability (low reactivity). In terms of the electronic nature of the molecule, aromaticity describes a conjugated system often made of alternating single and double bonds in a ring. This configuration allows for the electrons in the molecule's pi system to be delocalized around the ring, increasing the molecule's stability. The molecule cannot be represented by one structure, but rather a resonance hybrid of different structures, such as with the two resonance structures of benzene.

Conjugation is possible by means of alternating single and double bonds in which each atom supplies a p orbital perpendicular to the plane of the molecule. However, that is not the only way for conjugation to take place. As long as each contiguous atom in a chain has an available p orbital, the system can be considered conjugated. For example, furan is a five-membered ring with two alternating double bonds flanking an oxygen in a five-membered ring. Oxygen has two lone pairs, one of which occupies a p orbital perpendicular to the ring on that position, thereby maintaining the conjugation of that five-membered ring by overlap with the perpendicular p orbital on each of the adjacent carbon atoms. The other lone pair remains in plane and does not participate in conjugation.

In general, any sp² or sp-hybridized carbon or heteroatom, including ones bearing an empty orbital or lone pair orbital, can participate in conjugated systems, though lone pairs do not always participate in a conjugated system. For example, in pyridine, the nitrogen atom already participates in the conjugated system through a formal double bond with an adjacent carbon, so the lone pair remains in the plane of the ring in an sp² hybrid orbital and does not participate in the conjugation. A requirement for conjugation is orbital overlap; thus, the conjugated system must be planar (or nearly so). As a consequence, lone pairs which do participate in conjugated systems will occupy orbitals of pure p character instead of spⁿ hybrid orbitals typical for nonconjugated lone pairs.

In organic fluorescent structures, conjugated π systems absorb UV or visible light. Deletion of specific absorbed wavelengths from reflected visible light leads to the perception of color. However, a very small fraction of conjugated systems converts the absorbed energy into re-emission of light-fluorescence. Absorbance of light by a conjugated π system is the result of the energy of incoming UV and/or visible light matching the π/π* energy gap. This allows excitation of a HOMO π electron to the π* orbital (Prior to excitation, the π* orbital would be denoted as the LUMO.). This generates a high-energy (excited) state of the molecule, where one electron populates the antibonding π* orbital, and one electron remains in what was the fully-bonding π orbital.

The utility of fluorescence originates with the difference between the excitation and emission wavelengths. Because the excitation and emission wavelengths are different, emission intensity can be measured with minimized interference from the incoming excitation light, enabling to distinguish input and output.

Non-limiting examples of organic fluorescent cores that can be comprised by a probe for use in the context of the method of the invention comprise tetrapyrrole derivatives, such as porphyrin or phthalocyanine, acridine, BODIPY, cyanine or cyanine derivatives, carbazole, coumarin or coumarin derivatives, xanthene or xanthene derivatives such as fluorescein or rhodamine.

Tetrapyrroles are a class of chemical compounds that contain four pyrrole or pyrrole-like rings. The pyrrole/pyrrole derivatives are linked by (=(CH)- or -CH₂- units), in either a linear or a cyclic fashion. Pyrroles are a five-atom ring with four carbon atoms and one nitrogen atom. Tetrapyrroles are common cofactors in biochemistry and their biosynthesis and degradation feature prominently in the chemistry of life.

Porphyrin is a particularly preferred fluorescent core of the invention. Porphyrins are a group of heterocyclic macrocycle organic compounds, composed of four modified pyrrole subunits interconnected at their α carbon atoms via methine bridges (=CH-). The parent of porphyrin is porphine, a rare chemical compound of exclusively theoretical interest. Substituted porphines are called porphyrins and can be represented by the following formula:

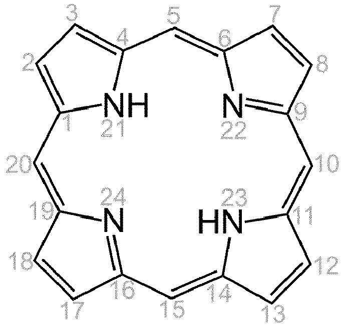

With a total of 26 π-electrons, of which 18 π-electrons form a planar, continuous cycle, the porphyrin ring structure is often described as aromatic. One result of the large conjugated system is that porphyrins typically absorb strongly in the visible region of the electromagnetic spectrum, i.e. they are deeply colored.

Phthalocyanine (H₂Pc) is a large, aromatic, macrocyclic, organic compound with the formula (C₈H₄N₂)₄H₂ and is of specialized interest. It can be depicted by the following formula:

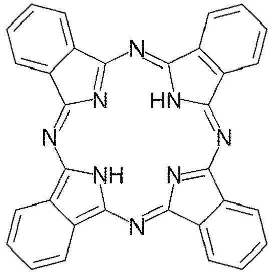

Phthalocyanine is composed of four isoindole units linked by nitrogen atoms. H₂Pc has a two-dimensional geometry and a ring system consisting of 18 π-electrons. The extensive delocalization of the π-electrons affords the molecule useful properties, lending itself to applications in dyes and pigments. There are many derivatives of the parent phthalocyanine, where either carbon atoms of the macrocycle are exchanged for nitrogen atoms or the peripheral hydrogen atoms are substituted by functional groups like halogens, hydroxyl, amine, alkyl, aryl, thiol, alkoxy and nitrosyl groups. These modifications allow for the tuning of the electrochemical properties of the molecule such as absorption and emission wavelengths and conductance.

It is understood that in the context of the invention a fluorescent core can be extended to comprise aromatic tethers that extend the conjugated electron system of the base structure of the organic core. For example, in embodiments the metal binding functional group can be linked to the fluorescent core via aromatic aryl-tethers, for example in form of an arylphsphonate or arylarsonate, such as phenylphosphonate or phenylarsonate. The metal binding functional group may be linked in ortho, meta or para position of the aryl/phenyl-ring. As show in the examples below, meta and para phenylphosphonic acid groups are both compatible with porphyrin as a core structure of the fluorescent core.

In embodiments, the organic fluorescent core of the probe is acridine or derivatives thereof. Acridine is an organic compound and a nitrogen heterocycle with the formula C₁₃H₉N.

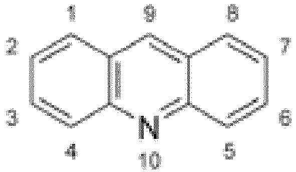

Acridines are substituted derivatives of the parent ring. It is a planar molecule that is structurally related to anthracene with one of the central CH groups replaced by nitrogen. Like the related molecules pyridine and quinoline, acridine is mildly basic. It is an almost colorless solid, which crystallizes in needles. There are several commercial applications of acridines, such as the use of acridine dyes, for example acridine orange (3,6-dimethylaminoacridine).

In embodiments, the organic fluorescent core of the probe is BODIPY or derivatives thereof. BODIPY is the technical common name of a chemical compound with formula C₉H₇BN₂F₂, whose molecule consists of a boron difluoride group BF₂ joined to a dipyrromethene group C₉H₇N₂; specifically, the compound 4,4-difluoro-4-bora-3a,4a-diaza-s-indacene in the IUPAC nomenclature. The common name is an abbreviation for "boron-dipyrromethene".

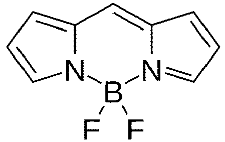

BODIPY is a red crystalline solid, stable at ambient temperature, soluble in methanol. Derivatives are obtained by replacing one or more hydrogen atoms by other functional groups and comprise the important class of BODIPY dyes. These organoboron compounds are often used as fluorescent dyes and markers in biological research.

In embodiments, the organic fluorescent core of the probe is cyanine or a cyanine derivative. Cyanines are synthetic dyes with the general formula
R2N[CH=CH]nCH=N+R2↔R2N+=CH[CH=CH]nNR2 (n is a small number) in which the nitrogen and part of the conjugated chain usually form part of a heterocyclic system, such as imidazole, pyridine, pyrrole, quinoline and thiazole. Cyanines are used in industry biotechnology (labeling, analysis, biomedical imaging).

In embodiments, the organic fluorescent core of the probe is carbazole. Carbazole is an aromatic heterocyclic organic compound. It has a tricyclic structure, consisting of two six-membered benzene rings fused on either side of a five-membered nitrogen-containing ring. The compound's structure is based on the indole structure, but in which a second benzene ring is fused onto the five-membered ring at the 2-3 position of indole (equivalent to the 9a-4a double bond in carbazole, respectively).

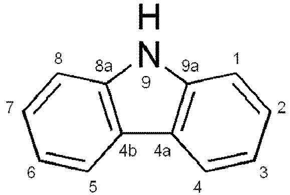

In embodiments, the organic fluorescent core of the probe is coumarin or a coumarin derivative. Coumarin or 2H-chromen-2-one is an aromatic organic chemical compound with the formula C9H6O2. Its molecule can be described as a benzene molecule with two adjacent hydrogen atoms replaced by a lactone-like chain -(CH)=(CH)-(C=O)-O-, forming a second six-membered heterocycle that shares two carbons with the benzene ring.

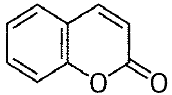

Coumarin can be placed in the benzopyrone chemical class and considered as a lactone. Coumarin and its derivatives are all considered phenylpropanoids. Some naturally occurring coumarin derivatives include umbelliferone (7-hydroxycoumarin), aesculetin (6,7-dihydroxycoumarin), herniarin (7-methoxycoumarin), psoralen and imperatorin. Compounds derived from coumarin are also called coumarins or coumarinoids; this family includes: brodifacoum, bromadiolone, difenacoum, auraptene, ensaculin, phenprocoumon (Marcoumar), PSB-SB-487, PSB-SB-1202, Scopoletin (can be isolated from the bark of Shorea pinanga), warfarin (Coumadin).

In embodiments, the organic fluorescent core of the probe is xanthene or xanthene derivatives such as fluorescein or rhodamine. Xanthene (9H-xanthene, 10H-9-oxaanthracene) is the organic compound with the formula CH₂[C₆H₄]₂O.

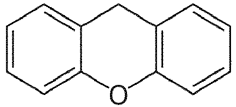

It is a yellow solid that is soluble in common organic solvents.

Many xanthene derivatives are useful dyes. Such xanthene dyes that contain a xanthene core include fluorescein 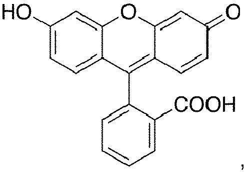 eosins, and rhodamines

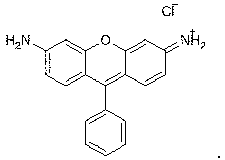

Xanthene dyes tend to be fluorescent, yellow to pink to bluish red, brilliant dyes. Many xanthene dyes can be prepared by condensation of derivates of phthalic anhydride with derivates of resorcinol or 3-aminophenol.

The term "metal binding functional group" relates to functional groups capable of binding to metal atoms, preferably metal ions. Accordingly, it is preferred that such functional groups are negatively charged acid groups that can bind metal cations. The functional groups of the probes of the invention are selected from the group comprising a phosphonic acid group and an arsonic acid group. In embodiments, the functional groups of the probes of the invention are selected from the group consisting of a phosphonic acid group and an arsonic acid group. In embodiments, the fluorescent probe comprises one or more phosphonic acid and/or arsonic acid groups.

Phosphonates and phosphonic acids are organophosphorus compounds containing -PO(OH)2, and phosphonicaciddiesters contain -PO(OR)2 groups (where R = alkyl, aryl). In the context of the invention, the C-atom connected to the P-Atom of the phosphonic acid is a sp or a sp²-carbon atom (C-PO(OH)2). Furthermore, in embodiments the -PO(OH)2 group may also be bound to a nitrogen atom of the fluorescent core. In embodiments, the nitrogen atom is an sp or an sp²-nitrogen atom (N-PO(OH)2).

Organophosphorus compounds are organic compounds containing phosphorus. Organophosphorus chemistry is the corresponding science of the properties and reactivity of organophosphorus compounds. Phosphorus, like nitrogen, is in group 15 of the periodic table, and thus phosphorus compounds and nitrogen compounds have many similar properties. According to one definition of organophosphorus compounds used herein, an organophosphorus compound need contain only an organic substituent, but need not have a direct phosphorus-carbon (P-C) bond. A large group of organophosphorus compounds is known to the skilled person. For example, phosphonates are esters of phosphonic acid and have the general formula RP(=O)(OR')2; phosphate esters have the general structure P(=O)(OR)3 feature P(V); Phosphine oxides (designation σ⁴λ⁵) have the general structure R₃P=O with formal oxidation state V; Compounds with the formula [PR₄⁺]X⁻ comprise the phosphonium salts; Phosphites, sometimes called phosphite esters, have the general structure P(OR)₃ with oxidation state +3; intermediate between phosphites and phosphines are phosphonites (P(OR)₂R') and phosphinite (P(OR)R_{'2}); the parent compound of the phosphines is PH3 or phosphane elsewhere, replacement of one or more hydrogen centers by an organic substituents (alkyl, aryl), gives PH3-xRx, an organophosphine, generally referred to as phosphines; compounds with carbon phosphorus(III) multiple bonds are called phosphaalkenes (R₂C=PR) and phosphaalkynes (RC≡P). Further example of organophohsphorus compounds are known to the skilled person.

Arsonic acids are a subset of organoarsenic compounds defined as oxyacids where a pentavalent arsenic atom is bonded to two hydroxyl groups, a third oxygen atom (this one with a double bond), and an organic substituent, which in the context of the present invention is either a C-atom (sp or sp²) or a N-atom (sp or sp²). The salts/conjugate bases of arsonic acids are called arsonates. Arsonic acid refers to H₃AsO₃, the case where the substituent is a single hydrogen atom. The other arsonic acids can simply be viewed as hydrocarbyl derivatives of this base case.

Methylarsonic acid results when the substituent is a methyl group. Phenylarsonic acid results when the substituent is a phenyl group.

The probes that can be used in the method of the invention comprise at least one metal binding functional group. Preferably, the probes comprise more than one metal binding functional groups, such as 2, 3, 4, 5, 6, 7, 8, 9, 10, 11, 12, 13, 14, 15, 16, 17, 18, 19, 20 or more.

The metal binding functional group is covalently linked to a sp or sp²-carbon atom or a nitrogen atom of the fluorescent core via a P or As atom.

Orbital hybridization (or hybridization) is the concept of mixing atomic orbitals into new hybrid orbitals (with different energies, shapes, etc., than the component atomic orbitals) suitable for the pairing of electrons to form chemical bonds in valence bond theory. Hybrid orbitals are very useful in the explanation of molecular geometry and atomic bonding properties and are symmetrically disposed in space. Orbitals are a model representation of the behavior of electrons within molecules. In the case of simple hybridization, this approximation is based on atomic orbitals, similar to those obtained for the hydrogen atom, the only neutral atom for which the Schrödinger equation can be solved exactly. In heavier atoms, such as carbon, nitrogen, and oxygen, the atomic orbitals used are the 2s and 2p orbitals, similar to excited state orbitals for hydrogen.

Hybrid orbitals are assumed to be mixtures of atomic orbitals, superimposed on each other in various proportions. For example, in methane, the C hybrid orbital which forms each carbon-hydrogen bond consists of 25% s character and 75% p character and is thus described as sp3 (read as s-p-three) hybridized.

Sp3-hybridization is explained for a tetrahedrally coordinated carbon (e.g., methane CH₄), the carbon should have 4 orbitals with the correct symmetry to bond to the 4 hydrogen atoms. Carbon's ground state configuration is 1s² 2s² 2p². The carbon atom can use its two singly occupied p-type orbitals, to form two covalent bonds with two hydrogen atoms, yielding the singlet methylene CH₂, the simplest carbene. The carbon atom can also bond to four hydrogen atoms by an excitation (or promotion) of an electron from the doubly occupied 2s orbital to the empty 2p orbital, producing four singly occupied orbitals. The energy released by the formation of two additional bonds more than compensates for the excitation energy required, energetically favoring the formation of four C-H bonds. Quantum mechanically, the lowest energy is obtained if the four bonds are equivalent, which requires that they are formed from equivalent orbitals on the carbon. A set of four equivalent orbitals can be obtained that are linear combinations of the valence-shell (core orbitals are almost never involved in bonding) s and p wave functions, which are the four sp³ hybrids. In CH₄, four sp3 hybrid orbitals are overlapped by hydrogen 1s orbitals, yielding four σ (sigma) bonds (that is, four single covalent bonds) of equal length and strength.

Sp²-hybridization can be explained in a similar way. For example, ethene (C2H4) has a double bond between the carbons. For this molecule, carbon sp² hybridizes, because one π (pi) bond is required for the double bond between the carbons and only three σ bonds are formed per carbon atom. In sp² hybridization the 2s orbital is mixed with only two of the three available 2p orbitals, usually denoted 2px and 2py. The third 2p orbital (2pz) remains unhybridized forming a total of three sp² orbitals with one remaining p orbital. In ethylene (ethene) the two carbon atoms form a σ bond by overlapping one sp² orbital from each carbon atom. The π bond between the carbon atoms perpendicular to the molecular plane is formed by 2p-2p overlap. Each carbon atom forms covalent C-H bonds with two hydrogens by s-sp² overlap, all with 120° bond angles. The hydrogen-carbon bonds are all equal strength and length, in agreement with experimental data.

The chemical bonding in compounds such as alkynes with triple bonds is explained by sp hybridization. In this model, the 2s orbital is mixed with only one of the three p orbitals, resulting in two sp orbitals and two remaining p orbitals. The chemical bonding in acetylene (ethyne) (C₂H₂) consists of sp-sp overlap between the two carbon atoms forming a σ bond and two additional π bonds formed by p-p overlap. Each carbon also bonds to hydrogen in a σ s-sp overlap at 180° angles.

Specific examples of fluorescent probes that can be used in the context of the present invention are provided herein.

For example, the probe may comprise porphyrin as an organic fluorescent core, wherein one or more of the sp²-carbon atoms are substituted with phosphonic acid or arsonic acid, wherein the P or As atom of the phosphonic acid or arsonic acid group are linked to the sp²-carbon atom. The phosphonic acid or arsonic acid group may be linked directly to the sp²-carbon of the porphyrin core, or via a suitable tether that extends the conjugated electron system of the core, such as a phenyl tether.

Preferred non-limiting examples of fluorescent probes for use in the method of the present invention in **Table 1** below.

In the displayed embodiments of **Table 1** comprise at least one -PO₃H₂, -AsO₃H₂, -R"PO>₃H₂ or-R"AsO₃H₂.

R' can be a hydrogen atom or -PC₃H₂, -AsO₃H₂, -R"PO₃H₂ and -R"AsO₃H₂ and their monoesters. In embodiments, R' can be a halogen atom (F, CI, Br, I) or an aryl or alkyl group or a functional group known in organic chemistry. In preferred embodiments, R' that are not -PO₃H₂, -AsO₃H₂, - R"PO₃H₂ or -R"AsO₃H₂ are H. In further preferred embodiments, R' that are not -PO₃H₂, -AsO₃H₂, -R"PO₃H₂ or -R"AsO₃H₂ are alkyl, such as preferably methyl, ethyl or other short alkyls with 1-6 C-atoms. Different R' can be the same or different within one of the disclosed formulas. The displayed embodiments of **Table 1** comprise at least one -PO₃H₂, -AsO₃H₂, -R"PO₃H₂ or-R"AsO₃H₂. If no -PO₃H₂, -AsO₃H₂, -R"PO₃H₂ or -R"AsO₃H₂ is specifically displayed, at least one R' is -PO₃H₂, -AsO₃H₂, -R"PO₃H₂ or -R"AsO₃H₂. In certain preferred embodiments, 2, 3, 4, 5, 6, 7 or 8 R' are -PO₃H₂, -AsO₃H₂, -R"PO₃H₂ or -R"AsO₃H₂.

R" is preferably a phenyl, biphenyl or triphenyl group or an aryl group, such as in the example phenylphosphonic acid. Preferably, different R" within one probe are the same. However, in embodiments, different R" within one molecule can also be different.

**Table 1. Preferred non-limiting examples of fluorescent probes for use in the method of the present invention.**

| **Structural formula of fluorascent probe** | **Name / Notes** | **Formula** |
|---|---|---|
| Fluorescent Phosphonic acids with phthalocyanine core: | | |
| 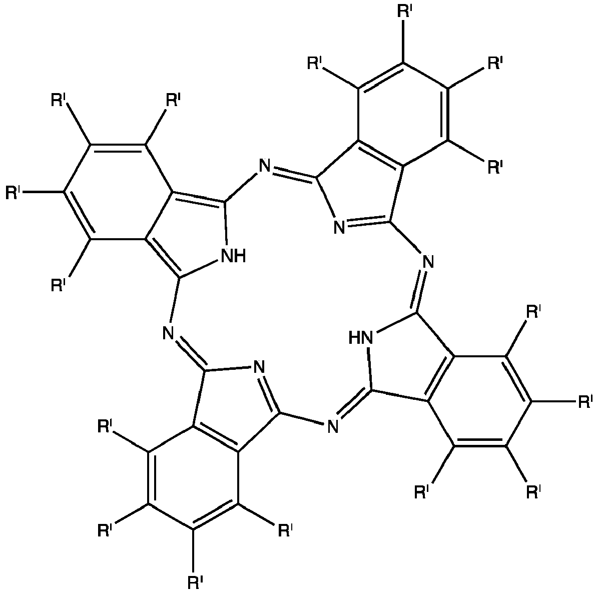 | | No. 1 |
| 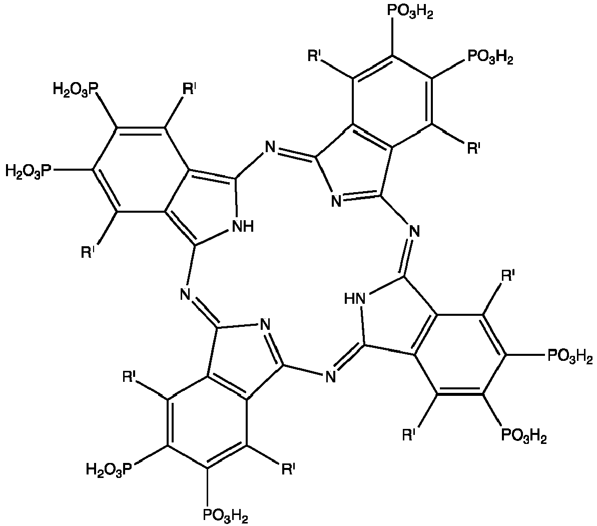 | Phthalocyanine hexaphosphonic acid | No. 2 |
| 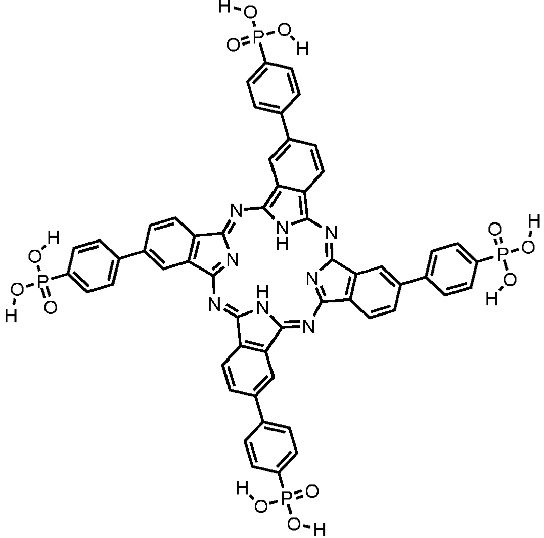 | Tetra phenylphosphonic acid tethered phthalocyanine | No. 3 |

| Fluorescent phosphonic acids with porphyrin core: | | |
|---|---|---|
| 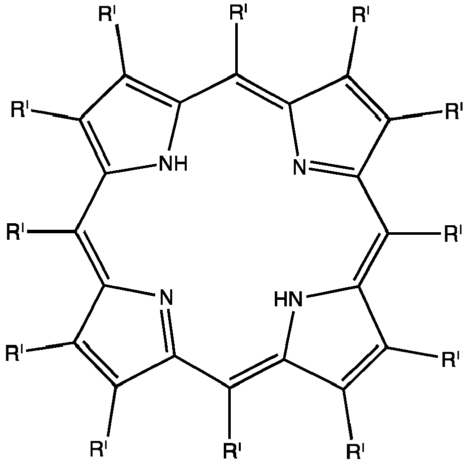 | | No. 4 |
| 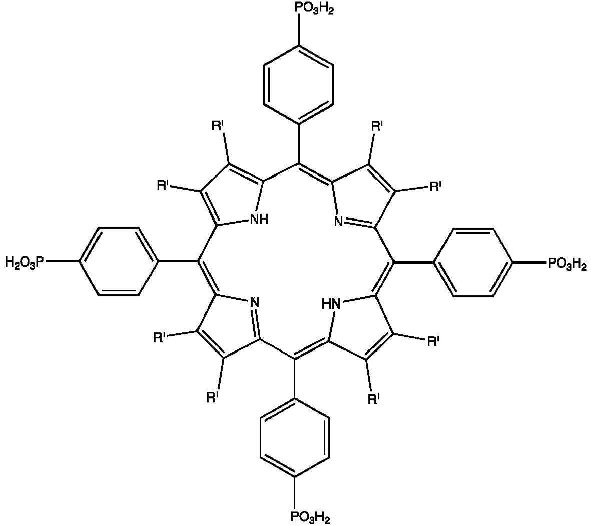 | Compound called *p*-H₈TPPA, when all R' are H. | No. 5 |
| 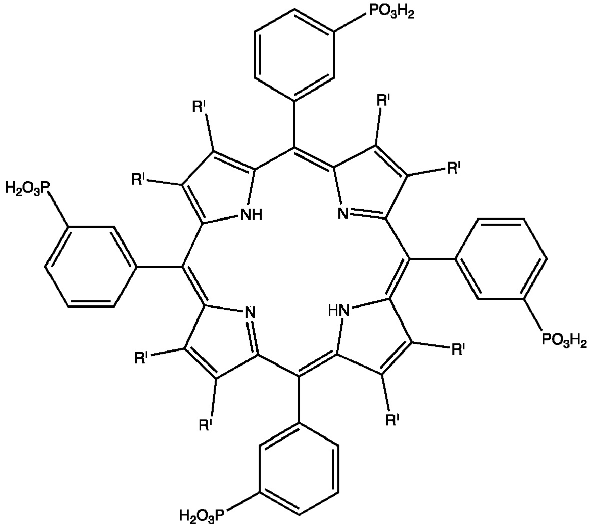 | Compound called *m*-H₈TPPA, when all R' are H. | No. 6 |
| 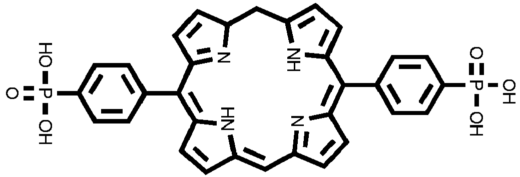 | 21*H*,23*H*-Porphine, 5, 15-bis(phenylphosphonic acid) | No. 7 |
| 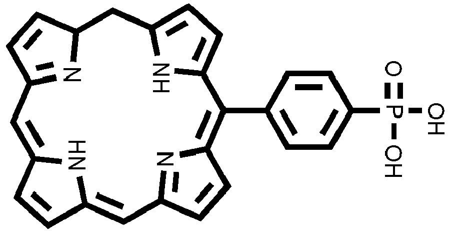 | 21*H*,23*H*-Porphine, 5-phenylphosphonic acid | No. 8 |
| 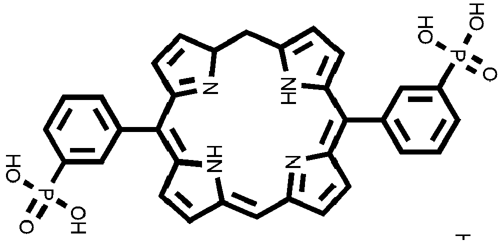 | 21*H*,23*H*-Porphine, 5, 15-bis(phenyl-3-phosphonic acid) | No. 9 |
| 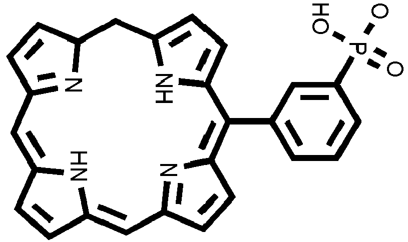 | 21*H*,23*H*-Porphine, 5-phenyl-3-phosphonic acid | No. 1 0 |
| 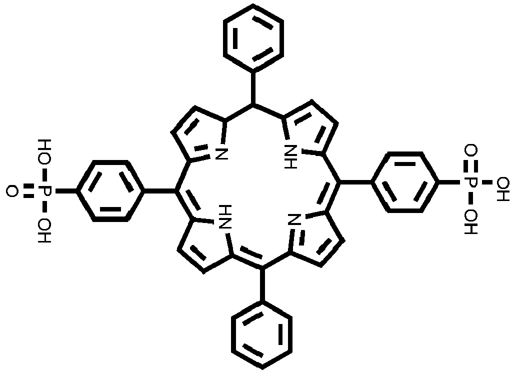 | 21*H*,23*H*-Porphine, 5, 15-bis(phenyl-4-phosphonic acid), 10, 20-bis(phenyl) | No. 11 |
| 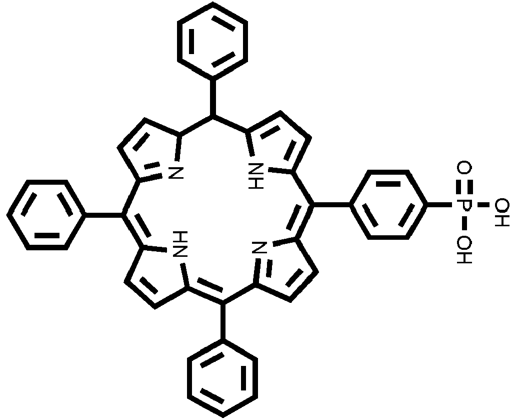 | 21*H*,23*H*-Porphine, 5-phenylphosphonic acid, 10, 15, 20-tris(phenyl) | No. 12 |

| Fluorescent phosphonic acids with coumarin core: | | |
|---|---|---|
| 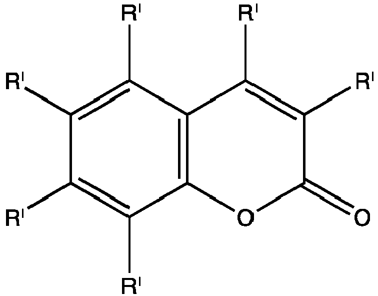 | | No. 13 |
| 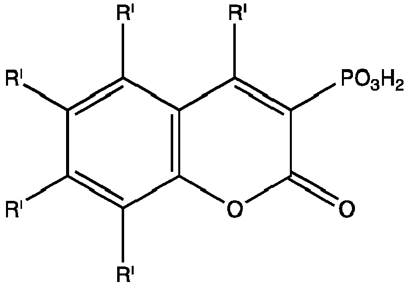 | In a preferred embodiment of No. 14, R' are H. Coumarin-3-phosphonic acid | No. 14 |
| 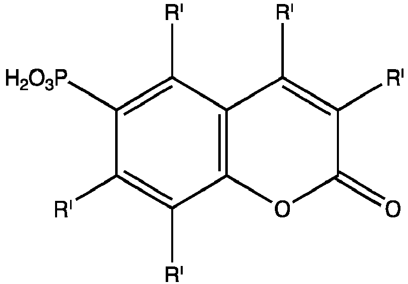 | Coumarin-6-phosphonic acid | No. 15 |

| Fluorescent phosphonic acids with rhodamine core: | | |
|---|---|---|
| 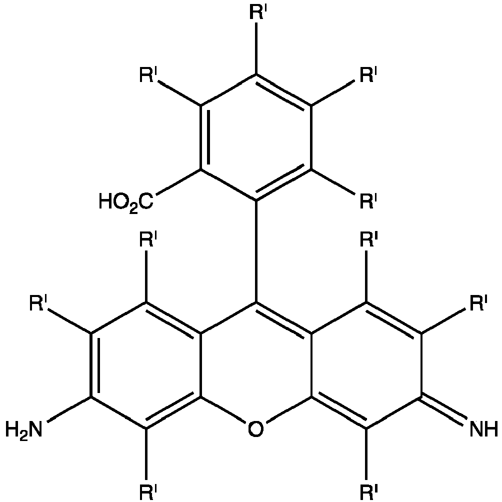 | | No. 16 |

| Fluorescent phosphonic acids with fluorescein core: | | |
|---|---|---|
| 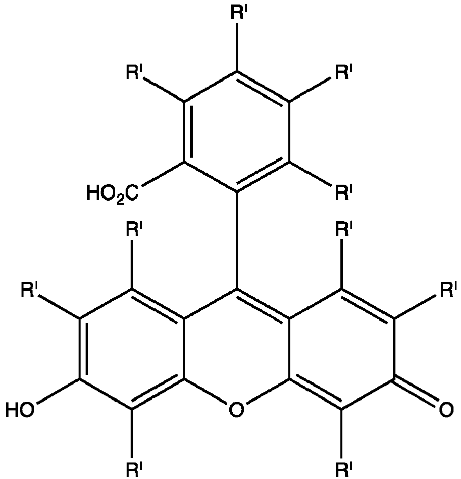 | | No. 17 |
| 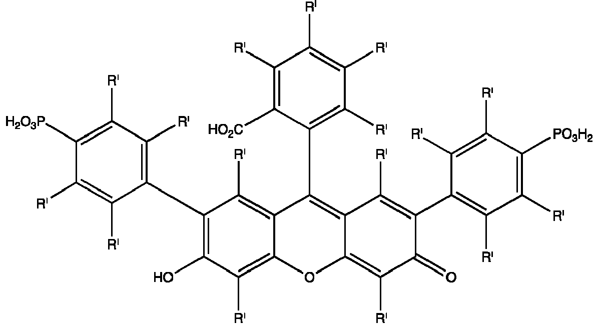 | Phenylphosphonic acid tethered fluorescein at the 2' and 7' positions 2',7'-fluoresceindiphenylphosphonic acid | No. 18 |
| 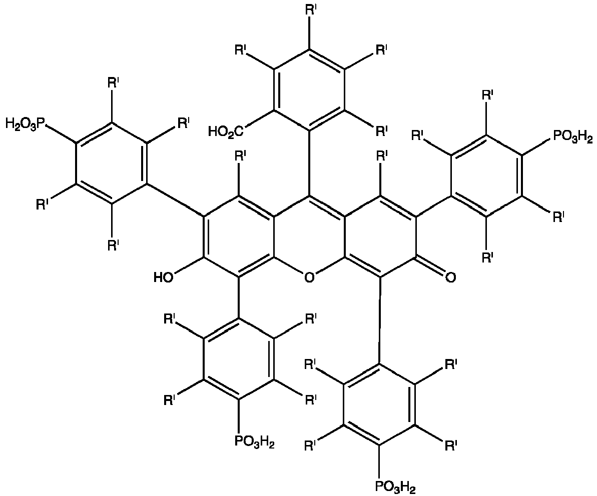 | Phenylphosphonic acid tethered fluorescein at the 2',4',5',7' positions 2',4',5',7'-fluoresceintetraphenylphosphonic acid | No. 19 |

| Fluorescent phosphonic acids with acridine core: | | |
|---|---|---|
| 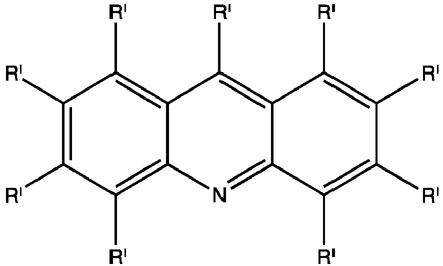 | | No. 20 |
| 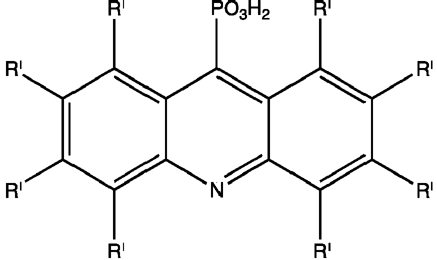 | Acridine-9-phosphonic acid | No. 21 |
| 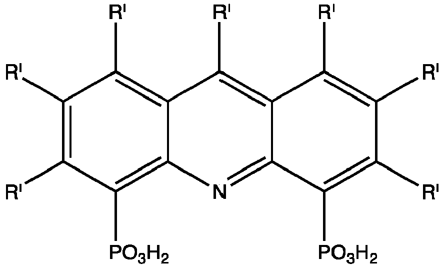 | 4,5-acridinediphosphonic acid | No. 22 |

| Fluorescent phosphonic acids with xanthene core: | | |
|---|---|---|
| 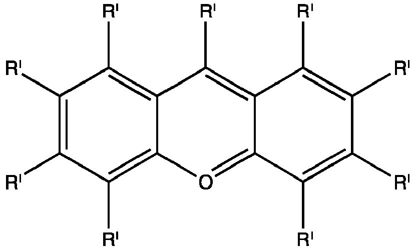 | | No. 23 |
| 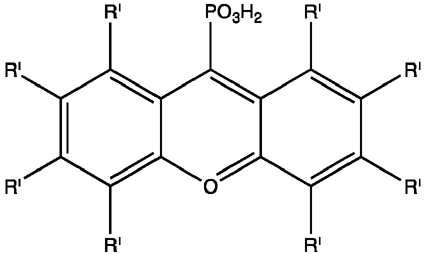 | Xanthene-9-phosphonic acid | No. 24 |
| 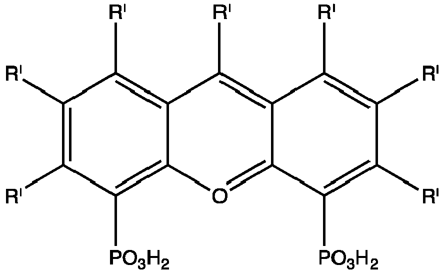 | 4,5-xanthenediphosphonic acid | No. 25 |

| Fluorescent phosphonic acids with carbazole core: | | |
|---|---|---|
| 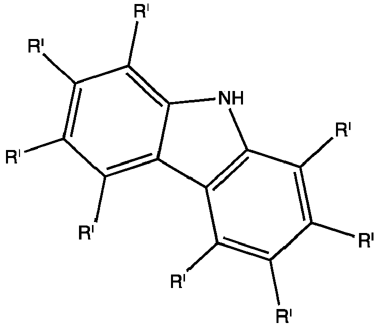 | | No. 26 |
| 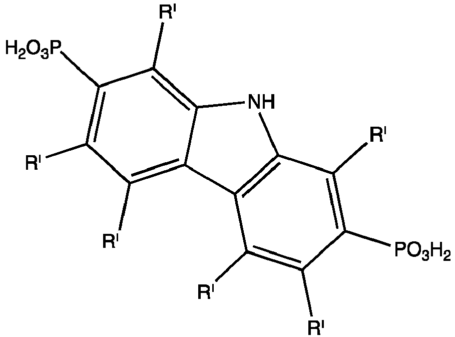 | 2,7-carbazolediphosphonic acid | No. 27 |

| Further examples of probes for use in the method of the invention: | | |
|---|---|---|
| 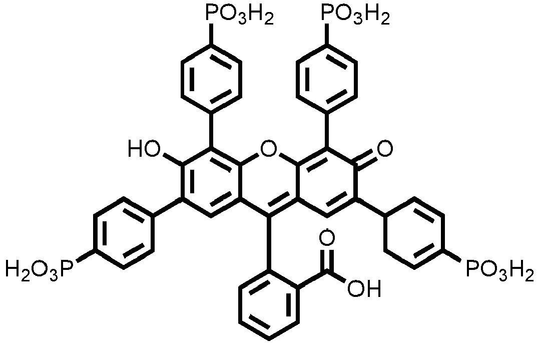 | | No. 28 |
| 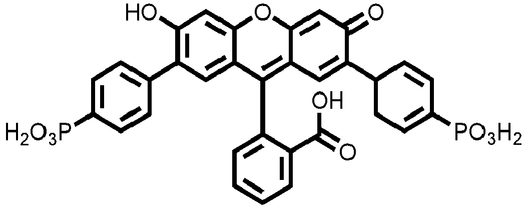 | | No. 29 |

It was found that presence of metal ions and binding of the metal ions by the metal binding functional groups of the probes of the invention leads to concentration-dependent changes of fluorescence of the probes. Such change may be a decrease, increase or shift of fluorescence compared to a reference sample. Therein, the reference sample can be a liquid sample comprising the same buffer or liquid as the sample of interest, but without any metal ions that can be detected by the probe. In embodiments, the detectable change of fluorescence is concentration dependent, meaning that for example a detectable decrease in fluorescence is more pronounced in the presences of a high amount/concentration of the respective metal ion as compared to samples comprising a lower amount/concentration of the same metal ion.

In embodiments of the invention, the concentration of free metal is calculated based on the mass action law, in particular involving calibration based on addition of a chelator and/or metal ions.

The mass action law (or law of mass action) is the proposition that the rate of a chemical reaction is directly proportional to the product of the activities or concentrations of the reactants. It explains and predicts behaviors of solutions in dynamic equilibrium. Specifically, it implies that for a chemical reaction mixture that is in equilibrium, the ratio between the concentration of reactants and products is constant.

In embodiments, the determining of a metal ion concentration requires quantification of the measured fluorescent signal. Such quantification may require comparison of the measured signal to a reference sample, which may be a sample known not to comprise metal ions of interest, and/or samples of known metal ion concentration and/or the sample of interest to which a metal chelator has been added in order to sequester the contained metal ions and prevent interaction of these metal ions with the probe. Such reference samples may also be referred to as calibration samples. In this context, the term calibration relates to the comparison of measured fluorescent signal generated from a sample of interest with the fluorescent signal of a calibration standard of known metal ion concentration.

Chelation is a type of bonding of ions and molecules to metal ions. It involves the formation or presence of two or more separate coordinate bonds between a polydentate (multiple bonded) ligand and a single central metal atom. These ligands are called chelators, but may also be referred to as chelants, chelating agents, or sequestering agents. Chelators are usually, but not necessarily, organic compounds. Common chelators that can also be used in the context of the present invention are EDTA and EGTA.

EDTA (Ethylenediaminetetraacetic acid) is an aminopolycarboxylic acid and a colourless, watersoluble solid. Its conjugate base is ethylenediaminetetraacetate. Its usefulness arises because of its role as a hexadentate ("six-toothed") ligand and chelating agent, i.e., its ability to sequester metal ions such as Ca2+ and Fe3+. After being bound by EDTA into a metal complex, metal ions remain in solution but exhibit diminished reactivity. EDTA is produced as several salts, notably disodium EDTA, calcium disodium EDTA, and tetrasodium EDTA (typically as the hydrate).

EGTA (ethylene glycol-bis(β-aminoethyl ether)-N,N,N',N'-tetraacetic acid), also known as egtazic acid (INN, USAN), is an aminopolycarboxylic acid, a chelating agent. It is a colourless solid that is related to the better known EDTA. Compared to EDTA, it has a lower affinity for magnesium, making it more selective for calcium ions. It is useful in buffer solutions that resemble the environment in living cells, where calcium ions are usually at least a thousand fold less concentrated than magnesium.

In embodiments of the invention, metal ion specific chelators can be added to the sample in order to sequester certain metal ions while others remain free and accessible for the fluorescent probe in solution.

In embodiments, the method of the invention is used in clinical diagnostics for determining available levels of free metal ions in a patient sample.

In embodiments, the invention relates to a method for diagnosis, prognosis, risk assessment, monitoring, therapy guidance and/or therapy control of a medical/clinical condition of a subject associated with changes in the level of metal ions in a bodily fluid or bodily sample. Such conditions comprise for example malnutrition, neurodegenerative diseases, such as Alzheimer's disease, Parkinson's disease and prion disease, and further disorders of metal metabolism, such as Wilson disease, Menkes disease, MEDNIK syndrome, Huppke-Brendel syndrome, hemochromatosis, neurodegeneration with brain iron accumulation, acrodermatitis enteropathica, transient neonatal zinc deficiency, spondylocheirodysplastic Ehlers-Danlos syndrome, Birk-Landau-Perez syndrome, hypermanganesemia with dystonia, SLC39A8 deficiency, SEPSECS deficiency, SBP2 deficiency.

The term "clinical diagnostics" or "diagnosis" relates to the recognition and (early) detection of a clinical condition of a subject. "Prognosis" relates to the prediction of an outcome or a specific risk for a subject. This may also include an estimation of the chance of recovery or the chance of an adverse outcome for said subject. In the present invention, the terms "risk assessment" and "risk stratification" relate to the grouping of subjects into different risk groups according to their further prognosis. Risk assessment also relates to stratification for applying preventive and/or therapeutic measures. The term "therapy stratification" in particular relates to grouping or classifying patients into different groups, such as risk groups or therapy groups that receive certain differential therapeutic measures depending on their classification. "Monitoring" relates to keeping track of an already diagnosed condition, disorder, complication or risk, e.g., to analyze the progression of the disease or the influence of a particular treatment or therapy on the disease progression of the disease in a patient. The term "therapy monitoring" or "therapy control" in the context of the present invention refers to the monitoring and/or adjustment of a therapeutic treatment of said subject, for example by obtaining feedback on the efficacy of the therapy. As used herein, the term "therapy guidance" refers to application of certain therapies, therapeutic actions or medical interventions based on the value/level of one or more biomarkers and/or clinical parameter and/or clinical scores, in particular the presence and concentration of metal ions in a patient sample. This includes the adjustment of a therapy or the discontinuation of a therapy.

In embodiments, the method of the invention is used in clinical diagnostics for determining available levels of free metal ions as a diagnostic marker for nutrient supply.

The instant disclosure also includes kits, packages and multi-container units containing the herein described reagents, such as the fluorescent probes and potentially buffer, chelators and/or other useful reagents for carrying out the method of the invention, and the use of such kits for performing the inventive method.

### FIGURES

The invention is further described by the following figures. These are not intended to limit the scope of the invention but represent preferred embodiments of aspects of the invention provided for greater illustration of the invention described herein.

### Description of the figures:

**Figure 1****:** *Metal-dependent changes of p*-H₈TPPA *absorbance and fluorescence properties.*
   10 µM *p*-H₈TPPA dilutions were treated with various metal solutions (final concentration 40 µM) before recording (A) absorbance, (B) fluorescence excitation (λ_{Em}. 650 nm) and (C) fluorescence emission (λ _{Exc.} 415 nm) spectra. Data are representative for three independent experiments.
**Figure 2****:** *Caco-2 fluorescence labeling with Phenylphosphonate-substituted porphyrines.*
   Caco-2 cells were incubated with increasing concentrations of *p*-H₈TPPA or *m*-H₈TPPA followed by fluorescence measurement (λ _{Exc/Em}. 416 nm/650 nm). Data are means ± S.E.M. of n=3 independent experiments.
**Figure 3****:** *Metal-dependent changes of m*-*H₈ TPPA absorbance and fluorescence properties.*
   10 µM *m*-H₈TPPA dilutions were treated with various metal solutions (final concentration 40 µM) before recording (A) absorbance, (B) fluorescence excitation (λ _{Em.} 650 nm) and (C) fluorescence emission (λ _{Exc.} 415 nm) spectra. Data are representative for three independent experiments.
**Figure 4****:** *3D fluorescence spectra of the phosphorylphenyl substituted porphyrins after metal cation treatment.*
   10 µM *p*-H₈TPPA (A) or *m*-H₈TPPA (B) dilutions were treated with either buffer or 40 µM CoCl₂, CdSO₄ or HgCl₂. A 3D scan was performed from 350 to 450 nm (Exc.) and 555 to 850 nm (Em.), with a 5 nm step size and excitation and emission bandwidths of 5 nm and a detector gain of 100.
**Figure 5****:** *Fluorescence labeling of Caco-2 enterocytes by phenylphosphonate-substituted porphyrines.*
   Fluorescence emission spectra of Caco-2 cells exposed to *p*-H₈TPPA, *m*-H₈TPPA (A) or the isopropyl-modified phosphonate *p*-H₈TPPA-iPr₈ (C) in comparison with each of the phenylphosphonate porphyrins as 10 µM dilution in assay buffer (λ_{Exc.} 416 nm). (B) Confocal images of Caco-2 upon loading with *p*-H₈TPPA or *m*-H₈TPPA. (D) Confocal images of *p*-H₈TPPA-iPr₈ in the presence or absence of Caco-2 cells. Scale bar = 10 µm. Data are representative for three independent experiments.

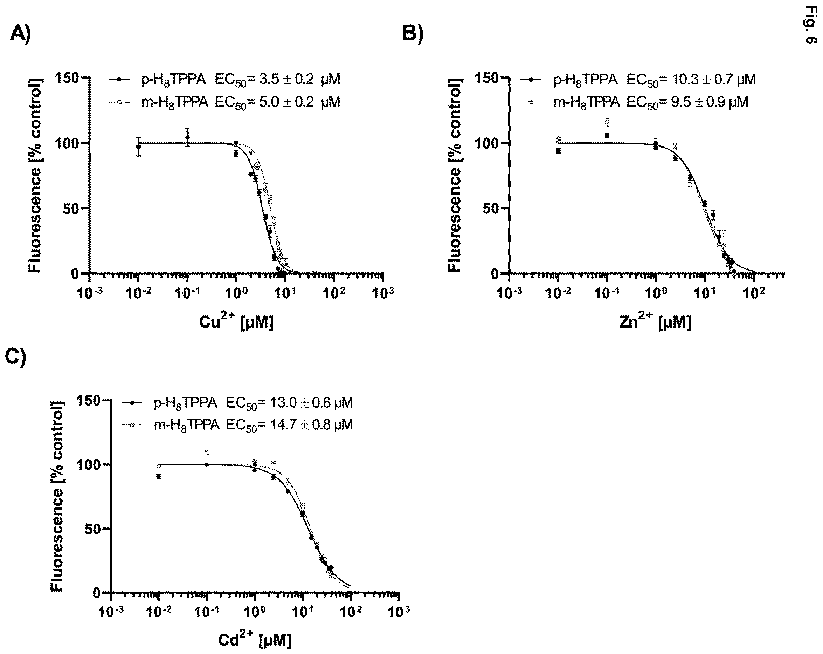

**Figure 6****:** *Fluorescence titration of p-H₈THPPA and m*-*H₈TPPA with metal cations.*
   10 µM *p*-H₈TPPA or *m*-H₈TPPA solutions were treated with increasing quantities of metal cations followed by detection of fluorescence emission λ_{Exc/Em.}416 nm/650 nm). Data are means ± S.E.M. of at least n=3 independent experiments. Sigmoidal dose-response curves were fitted by non-linear regression and the resulting EC₅₀ values are indicated.

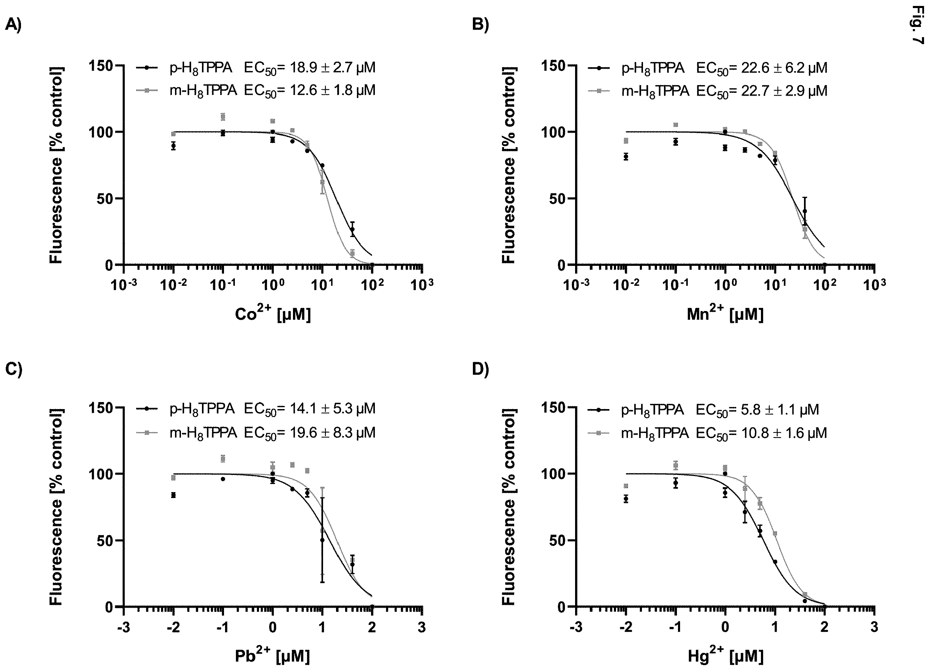

**Figure 7****:** *Fluorescence titration of p-H₈THPPA and m*-H₈*TPPA upon addition of metal cations*
   10 µM *p*-H₈TPPA or *p*-H₈TPPA solutions were treated with increasing quantities of metal cations followed by detection of fluorescence emission (λ_{Exc/Em.} 416 nm/650 nm). Data are means + S.E.M. of at least n=3 independent experiments. Sigmoidal dose-response curves were fitted by non-linear regression and the resulting EC₅₀ values are indicated.

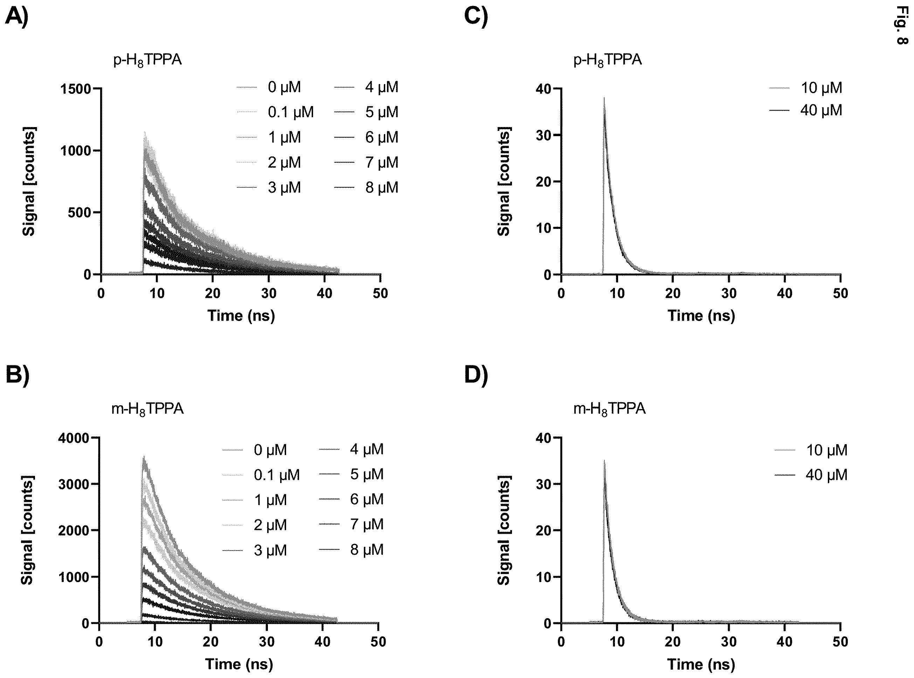

**Figure 8****:** *Fluorescence decay curves of p-H₈THPPA and m*-*H₈TPPA in the presence of CuSO₄.*
   Time-resolved fluorescence was measured on *p*-H₈THPPA and *m*-H₈TPPA (each 10 µM) in the presence of various amounts of CuSO₄ between 0 and 40 µM. CuSO₄ was admixed until the actual cupric concentration was reached. Then samples were gently mixed and incubated for 60 seconds before performing time-correlated single photon counting. For 10 and 40 µM CuSO₄ (C+D) the intensity of the excitation intensity was enhanced by a factor of 8 and measuring time was prolonged by a factor of 10 to improve signal/noise output. Data shown in C+D are scaled differently for direct comparison. Data are representative for one out of three independent experiments.

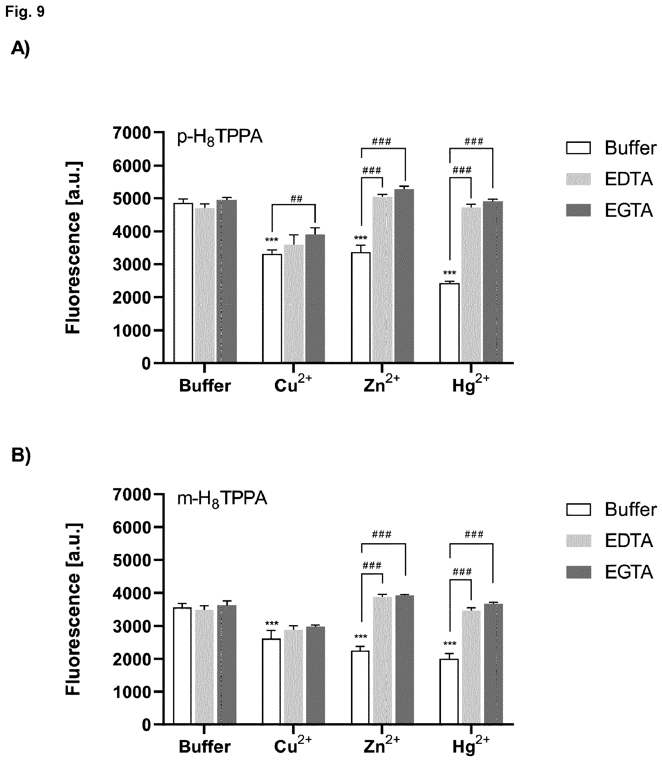

**Figure 9****:** *Reversibility of p-H₈THPPA and m*-H₈TPPA *metal complexation.*
   Fluorescence of metal cation pretreated *p*-H₈THPPA or m-H₈TPPA solutions upon addition of 50 equivalents of EDTA or EGTA (λ _{Exc/Em.} 416 nm/650 nm). Data are means ± S.E.M. of n=3 independent experiments. Statistically significant differences from buffer incubation (***p<0.001; two-way ANOVA/ Tukey post hoc test) or from metal cation treatment (##p< 0.01; ###p<0.001; two-way ANOVA/ Tukey post hoc test) are indicated.

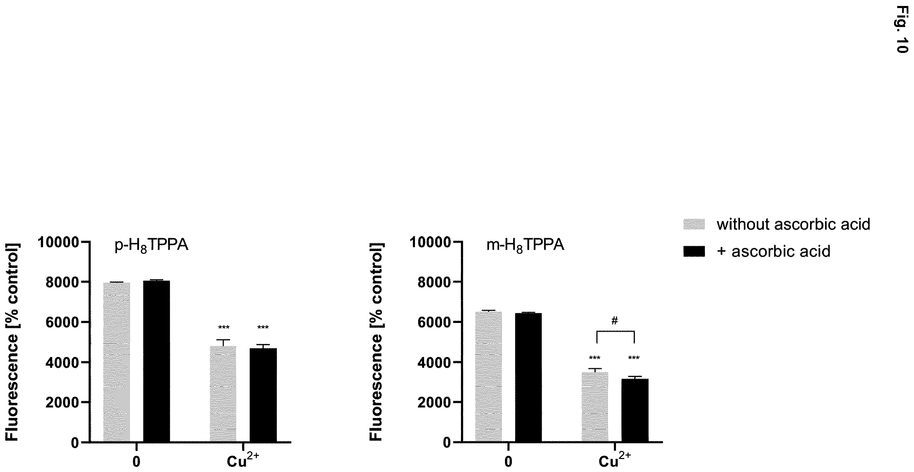

**Figure 10****:** *Effect of Cu-redox conversion on p-H₈THPPA and m*-H₈TPPA *fluorescence properties*
   *10 µM CuSO₄ solutions pretreated* +/- *500 µM of l-ascorbic acid (15 min) were mixed with p-H₈*-*TPPA or m*-H₈TPPA *followed by detection of fluorescence emission (*λ _{*Exc*/}*_{Em.} 416 nm*/*650 nm). Data are means* + *S.E.M. of n=3 independent experiments. Statistically significant differences from control ***p<0.001; two-way ANOVA*/ *Tukey post hoc test) or from Cu*^{*2*+} *alone (#p*<*0.05; two-way ANOVA*/ *Tukey post hoc test) are indicated.*

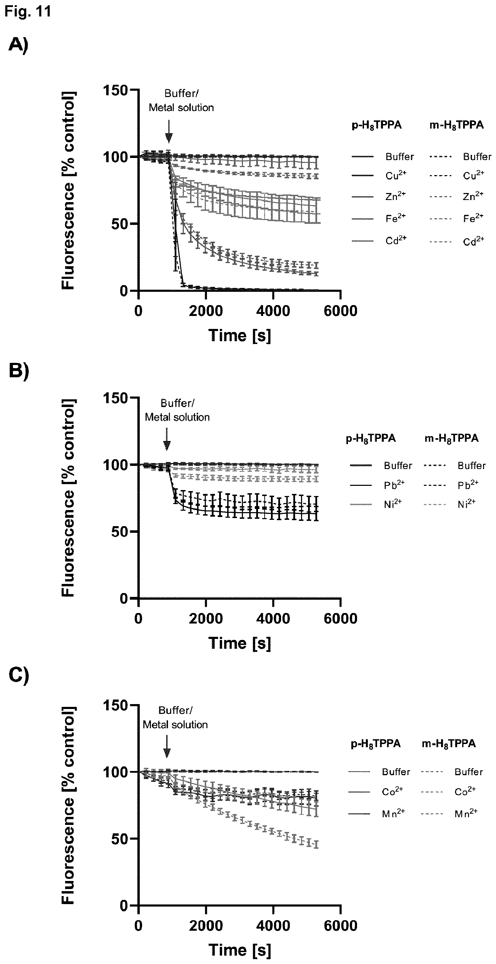

**Figure 11****:** *Live-cell sensing of metal uptake into Caco-2 enterocytes with p-H₈THPPA and m-H₈TPPA.*
   Caco-2 cells were loaded with *p*-H₈THPPA or m-H₈TPPA and the fluorescence recorded in 3 min intervals (λ _{Exc/Em.} 416 nm/650 nm). Fifteen minutes after the start of the experiment (arrow), metal cation solutions (final concentration 50 µM) or buffer (control) were added and the fluorescence measurement continued. Data are means ± S.E.M. of n=3 independent experiments.

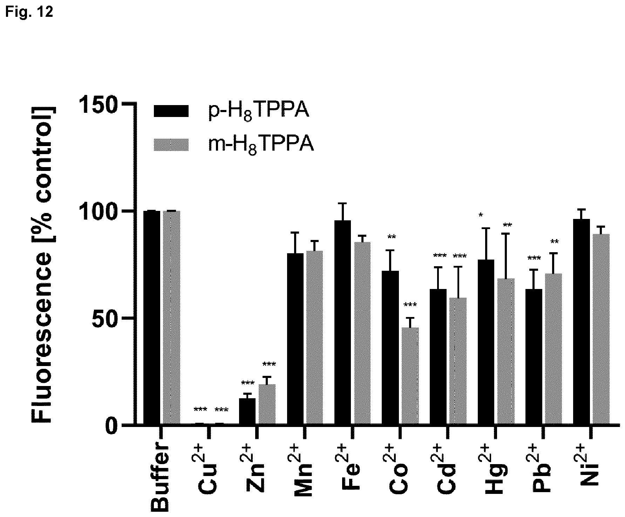

**Figure 12****:** *Summary of cellular p*-H₈TPPA *and m*-H₈TPPA *metal responsiveness.*
   Data were taken from the last fluorescence readout shown in Fig. 11. Data are means + S.E.M. of n=3 independent experiments. Significant differences from buffer incubation ***p<0.001; **p<0.01; *p<0.05; one-way ANOVA/ Dunnett's multiple comparison test.

### EXAMPLES

The invention is further described by the following examples. These are not intended to limit the scope of the invention but represent preferred embodiments of aspects of the invention provided for greater illustration of the invention described herein.

To examine a suspected metal-induced fluorescence change we used arylphosphonic acids namely, 5,10,15,20-tetrakis [*m*-phenylphosphonic acid] porphyrin **(*m*-H₈TPPA),** its positional isomer 5,10,15,20-Tetrakis [*p*-phenylphosphonic acid] porphyrin **(*p*-H₈TPPA),** and the ester form 5,10,15,20-tetrakis[*p*-(diisopropoxyphosphoryl)phenyl] porphyrin **(*p*-H₈TPPA-iPr₈);** to create a differentially altered fluorescence upon binding to metal ions. For this study, we have selected a range of biologically relevant metal ions and toxic heavy metals. We have observed a wide variety of unique fluorescent responses upon binding of the fluorophores with the biologically relevant metal ions Zn, Cu, Co, Ni, Fe, Mn, Mg, Ca, and toxic metal ions Cd, Pb, Hg, and Co in Caco-2 cells. We intentionally inhibited the extended conjugation of the fluorescent core **(*p-*H₈TPPA)** and the metal ion binding by using the isopropyl diester **(*p*-H₈TPPA-iPr₈)**, which showed no fluorescence change upon metal binding.

### Summary of the examples

We report the application of a highly versatile and engineerable novel sensor platform to monitor biologically significant and toxic metal ions in live human Caco-2 enterocytes. The extended conjugation between the fluorescent porphyrin core and metal ions via aromatic phenylphosphonic acid tethers generates a unique turn off and turn on fluorescence and, in addition, shifts in absorption and emission spectra for zinc, cobalt, cadmium and mercury. The reported fluorescent probes *p*-H₈TPPA and *m*-H₈TPPA can monitor a wide range of metal ion concentrations via fluorescence titration and also via fluorescence decay curves. Cu and Zn-induced turn off fluorescence can be differentially reversed by the addition of common chelators. Both *p*-H₈TPPA and *m*-H₈TPPA readily pass the mammalian cellular membrane due to their amphipathic character as confirmed by confocal microscopic imaging of living enterocytes.

### Results of the examples

### Metal-dependent changes of p-H₈TPPA absorbance and fluorescence properties.

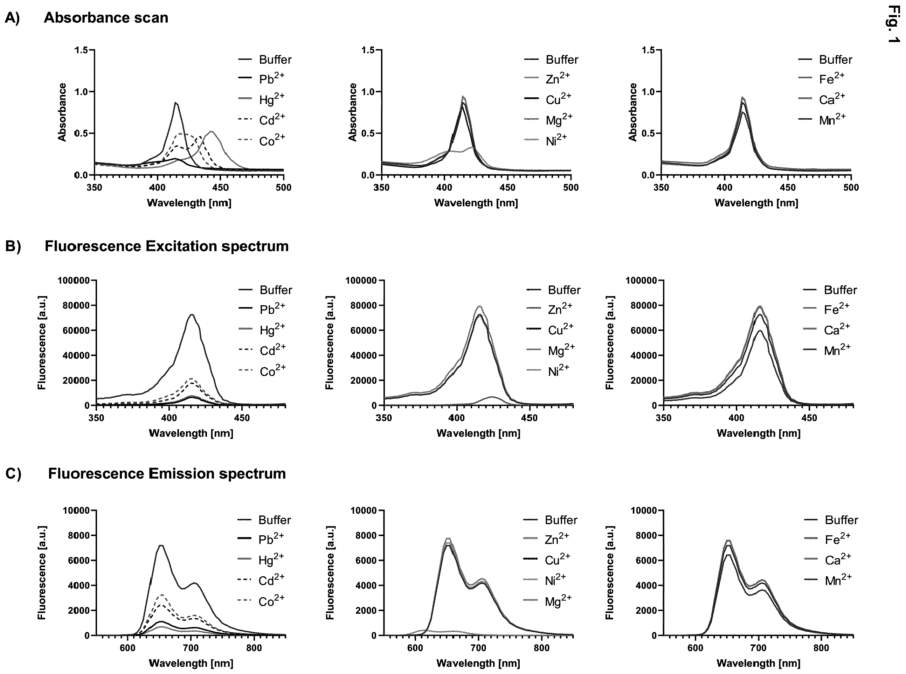

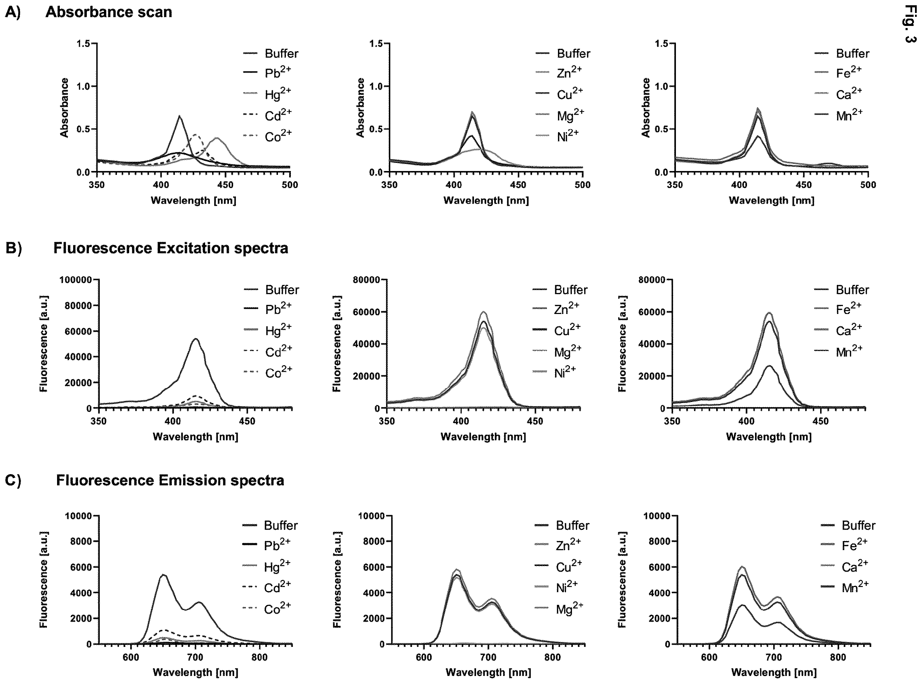

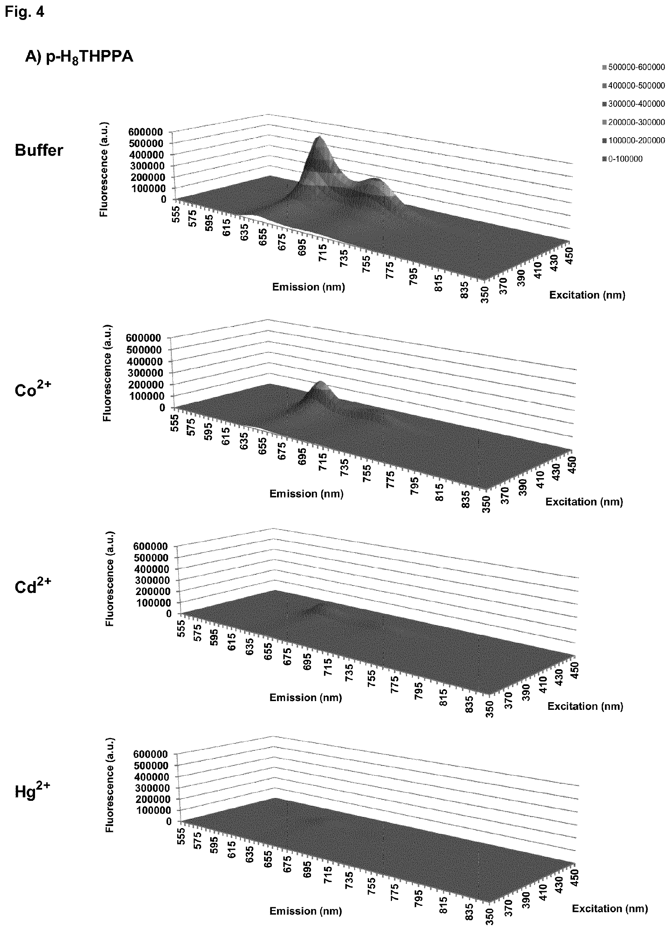

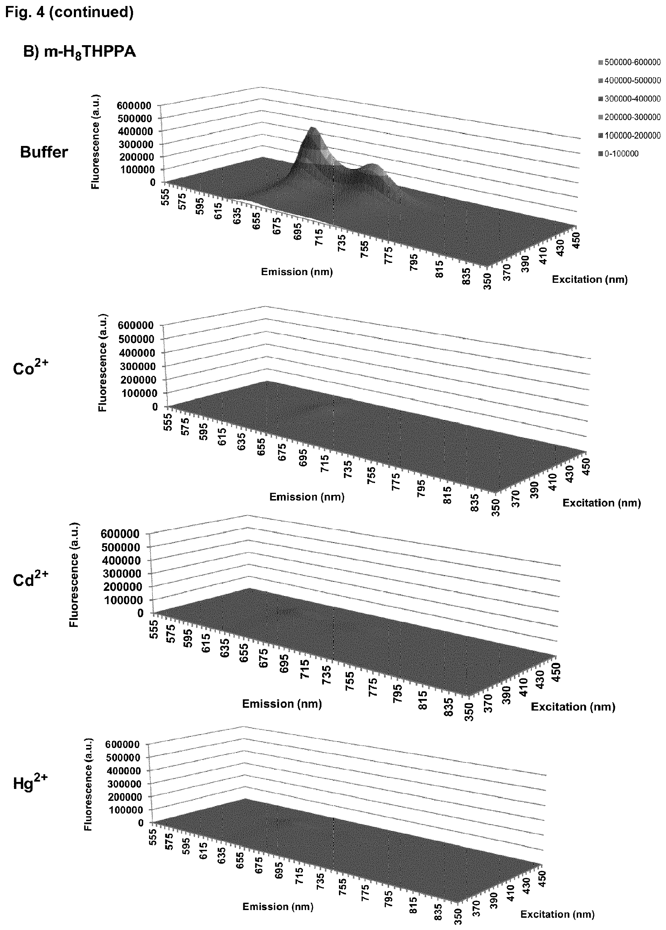

Aiming to use arylphosphonate-tethered porphyrins as real-time cellular metal fluorescence sensors, we first characterized their metal-responsiveness under cell-free conditions. *p*-H₈TPPA (see Fig. 1) and *m*-H₈TPPA (see Fig. 3), were pretreated with various bivalent metal ions at a pH close to the cellular milieu (pH 7.4) before recording absorption and fluorescence spectra. The visible absorption of both sensors in the buffer-control treatment showed maxima in the range of 380-430 nm, with a typical Soret peak at around 416 nm. Ca²⁺or Mg²⁺ treatment did not shift the position of the Soret maximum, but slightly raised the intensity of the absorption spectrum causing a higher fluorescence, characteristic for non-ratiometric metal-ON fluorescence sensors. Mn²⁺, and far more pronounced Pb²⁺, narrowed non-ratiometrically the photon absorbing properties of *p*-H₈TPPA and m-H₈TPPA, thus their fluorescence dropped markedly. The Cd²⁺-treated sensors showed a decline of the 416 nm absorbance maximum and the appearance of a new band peaking at 434 nm. Following Co²⁺ treatment absorption spectra were less intense, plateau-shaped with a bathochromic broadening up to 434 nm. Hg²⁺-treatment red-shifted the Soret band by approximately 30 nm. Albeit that, none of the absorption shifts led to comparable shifts in the emission bands. Despite of their restricted fluorescence intensity, the fluorescence emission patterns of all the aforementioned metal-treated arylphosphonate-tethered porphyrins seemed to be completely distinct und almost indistinguishable from the buffer-controls (see Fig. 1C, Fig. 2 and 4). Zn²⁺ treatment led to a declined and much more broadened Soret band in the range of 380-430 nm (see Fig. 1A). Still, the fluorescence emission was much lower than what would have been expected from the fluorescence excitation properties (see Fig. 1B and C). Thus, we assume a fluorescence quenching effect of Zn²⁺ that requires further investigation.

### Fluorescence labeling of Caco-2 enterocytes by phenylphosphonate-substituted porphyrines.

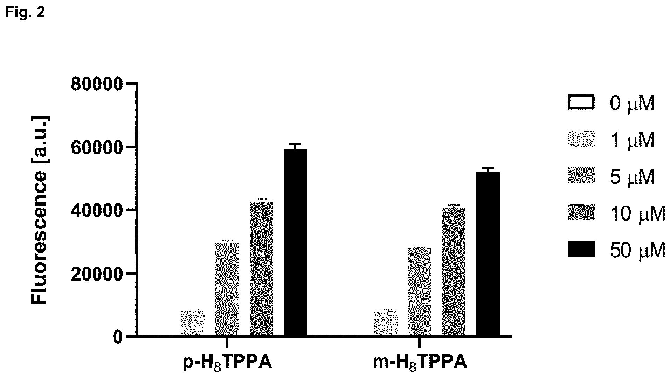

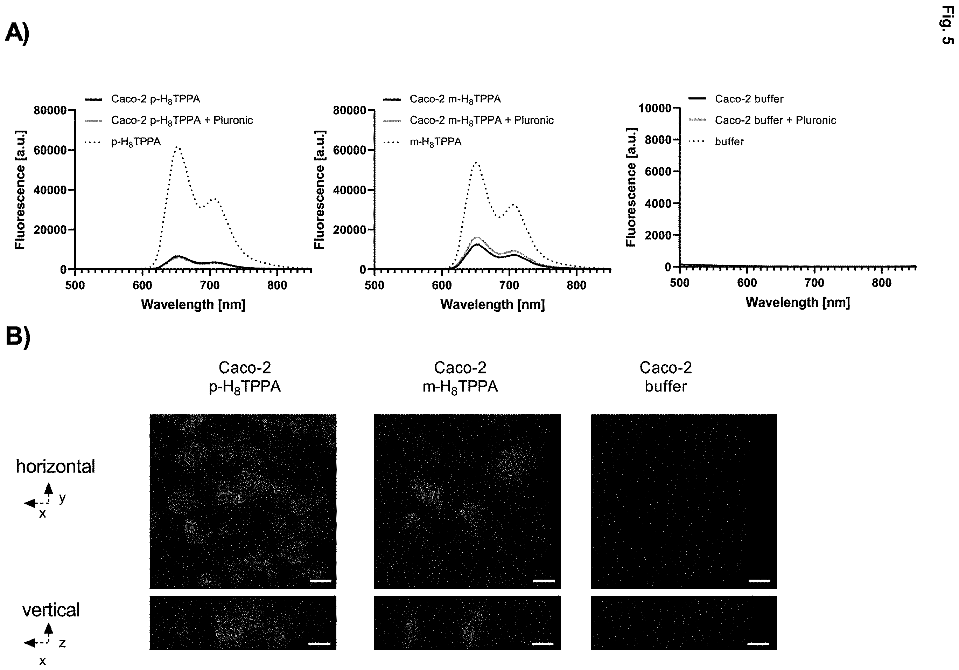

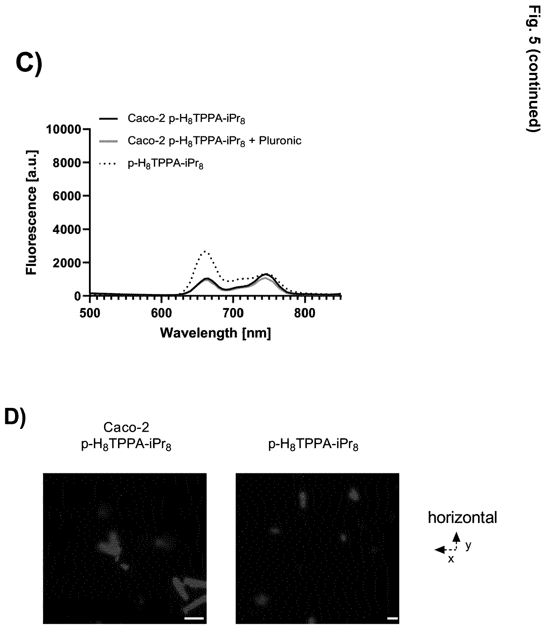

Steady-state fluorescence spectra of *p*-H₈TPPA/*m*-H₈TPPA-loaded Caco-2 cells exhibit a S1-fluorescence pattern with two-band emission maxima at 655 and 705 nm, almost perfectly matching the fluorescence characteristics of the sensor applied in a physiological buffer (see Fig. 5A). Thus, *p*-H₈TPPA and *m*-H₈TPPA seemed to pass the cellular membrane reaching the cell interior intact (see Fig. 5A and Fig. 2), a result further confirmed by confocal microscopic imaging of living enterocytes (see Fig. 5B).

We further performed cellular uptake experiments implementing the detergent pluronic F-127, aiming to counterbalance the partial hydrophobic properties of the porphyrin skeleton. Yet, the amphipathic character of *p*-H₈TPPA and m-H₈TPPA is sufficient to enable cellular entrance (see Fig. 5A). Aside from the two fluorescent arylphosphonic acids an isopropyldiester-modified variant (*p*-H₈TPPA-iPr₈), intentionally blocked for metal binding at the phosphonate moiety, was aimed for Caco-2 cell application. As seen in Fig. 5C, the fluorescence emission output of *p*-H₈TPPA-iPr was much lower in the incubation buffer as well as in the presence of cells. The confocal microscopy pictures depict *p*-H₈TPPA-iPr₈ microcrystals of ∼ 20 µm size, inaccessible for cellular uptake. Even the presence of the detergent pluronic did not improve the solubility of the diester in aqueous media (see Fig. 5C).

### Fluorescence titration of p-H₈THPPA and m-H₈TPPA with metal cations

Addition of CuSO₄ caused the most pronounced effects of all metal ions tested. Upon treatment with 40 µM Cu²⁺ almost complete abrogation of *p*-H₈TPPA and m-H₈TPPA (final concentrations 10 µM) fluorescence was observed (see Fig. 1C, Fig. 6A). The half maximal effective concentration for *p*/*m*-H₈TPPA fluorescence silencing was considerably lower for copper than for all the other metal cations tested (see Fig. 6 and Fig. 7).

### Time-resolved fluorescence spectroscopy

Time-resolved fluorescence spectroscopy was shown to be highly efficient unraveling interaction mechanisms between fluorescent probes and their surroundings, including specific ions ^{36,37}. It was successfully applied to unravel structural molecular changes that are correlated with fluorescence quenching ^{38,39}. In particular, it allows the quantitative study of dynamic electronic interactions between the fluorescent core and the metal ion to cause a characteristic change in fluorescence intensity and lifetime ⁴⁰⁻⁴².

The time-resolved fluorescence showed a dose-dependency in the initial amplitudes of the p-H₈TPPA fluorescence decay curves for Cu²⁺ at concentrations between 0 and 8 µM (Fig. 8A). Noticeably, the time constant was stable for all concentrations. The quantitative fit of the decay curves resulted in 8.3 ns fluorescence lifetime for *p*-H₈THPPA up to 8 µM copper dosing, so at low concentrations of CuSO₄ the observed quenching is purely static. At higher Cu²⁺ concentrations (10-40 µM) an increased excitation intensity and prolonged measuring time was needed to improve the signal-to-noise ratio. Under these conditions the copper quenching becomes dynamic (lifetime dropped to 1.7 ns starting at a concentration of CuSO₄ of 10 µM) and/or it is correlated with a molecular change (e.g., oxidation of the molecules), which induces the change of the fluorescence lifetime observed in Fig. 8C. Similar behavior was observed for *m-*H₈TPPA, which exhibited a constant lifetime of 7.7 ns up to 8 µM CuSO₄ with concomitant drop of the amplitude (Fig. 8B), while above this concentration a pronounced drop in lifetime was observed, resulting in 1.6 ns fluorescence decay time (Fig. 8D).

### Reversibility of p-H₈THPPA and m-H₈TPPA metal complexation

The assumption of an at least partially irreversible molecule change was further supported by the observation that the copper-induced decrease in *p*/*m*-H₈TPPA fluorescence remained almost non-responsive to metal chelator (EDTA or EGTA) posttreatment, whereas for Zn²⁺ as well as Hg²⁺ quenching was reversed by both chelators (Fig. 9). Quenching of *p*-H₈TPPA and *m*-H₈TPPA fluorescence was observed for Cu²⁺ and Cu⁺, irrespective of the oxidation state of copper (Fig. 10). This is of importance for future applications, given that copper is present as Cu⁺ in the intracellular milieu, whereas the cupric state (Cu²⁺) predominates in the systemic blood circulation 9,43

### Live-cell sensing of metal uptake into Caco-2 enterocytes

When present within Caco-2 cells, *p*-H₈TPPA/*m*-H₈TPPA fluorescence remained stable upon extracellular addition of 50 µM FeSO₄, whereas *in vitro* both sensors were slightly enhanced by iron treatment (Fig. 1). For Cd²⁺, Cu²⁺, Hg²⁺ Mn²⁺, Pb²⁺ and Zn²⁺ a 50 µM incubation concentration was already shown to be sufficiently high to admit quantitative metal ion delivery into Caco-2 ⁴⁴⁻⁴⁶. The arylphosphonate-tethered porphyrin sensors introduced into the human enterocytes record these incoming metal cations in a fluorescence silencing language (Fig. 11A-C, Fig. 12). The strong and rapid decline in Caco-2 *p*-H₈TPPA/*m*-H₈TPPA fluorescence upon Cu-exposure was somewhat surprising (Fig. 11A) keeping in mind the tight copper buffering ability of the cellular metallochaperons Atox1 (Antioxidant 1 Copper Chaperone), Ccs (Copper chaperone for superoxide dismutase), Cox17 (Cytochrome c oxidase copper chaperone) and glutathione (intracellular GSH concentration around 10 mM), balancing the pool of cellular labile copper in the femtomolar range ^{9,47}. It can be hypothesized that cellular homeostasis was overburdened by a supraphysiological amount of copper and therefore unable to ensure its correct sequestration.

Alternatively, the phenylphosphonic acid porphyrin sensors might detect intracellular protein-complexed copper in addition to free Cu⁺ ions. The same applies to Zn (Fig. 11A). The free intracellular zinc concentration estimated with the fluorescent sensor Zinpyr-1 in Caco-2 cells treated with 50 µM ZnSO₄ was approximately 2nM ⁴⁸. This is several orders of magnitude lower than the concentration required for fluorescence quenching of the phosphonate porphyrins *in vitro* (Fig. 6B).

### Discussion of the examples

Irrespective of the actual species causing *p*-H₈TPPA/*m*-H₈TPPA quenching, these probes are promising tools to monitor alterations of essential and toxic metals in human body fluids, cell and tissue samples during various life stages and in disease. Our design strategy of extending the conjugation of fluorescent core via sp² bonded phosphonic acids was successful in generating unique metal-responsive fluorescent behavior for each of the studied metal ions. Therefore, as a new class of non-toxic fluorophores, phenylphosphonic acid-functionalized porphyrins provide an expandable and engineerable platform for the development of improved, targeted fluorescence sensors suitable for *in vivo* applications to determine and visualize metals in tissues during disease progression. This is of utmost importance for diagnostics and in the development and translation of therapeutics for heavy metal intoxication as well as diseases associated with alterations in the homeostasis of essential metal ions.

### Materials and Methods of the examples

### Reagents

I-Ascorbic acid (Honeywell Riedel-de-Haën, Seelze, Germany); CaCl₂ (Sigma Aldrich, Munich, Germany); CdCl₂·H₂O (Honeywell Fluka^{™}, Seelze, Germany); Chelex^{®} 100 Resin (Bio-Rad, Hercules, CA, USA); CoCl₂·6H₂O (Honeywell Riedel-de-Haën, Seelze, Germany); CuSO₄ (Sigma Aldrich, Munich, Germany); Dulbecco's modified Eagles medium (DMEM) (PAN-Biotech, Aidenbach, Germany); EGTA (Honeywell Fluka^{™}, Seelze, Germany); FeSC₄·7H₂O (Merck-Millipore, Darmstadt, Germany); Fe₂(SO₄)₃ (Sigma Aldrich, Munich, Germany); fetal calf serum (FCS) (CCPro, Oberdorla, Germany); HgCl₂ (Merck-Millipore, Darmstadt, Germany); MgCl₂·6H₂O (Honeywell Riedel-de-Haën, Seelze, Germany); MnSO₄·H₂O (Carl Roth, Karlsruhe; Germany); Na₂EDTA·2H₂O (Carl Roth, Karlsruhe; Germany); Pb(NO₃)₂ (Sigma Aldrich, Munich, Germany); Penicillin-Streptomycin solution (Sigma Aldrich, Munich, Germany); ZnSO₄·7H₂O (Sigma Aldrich, Munich, Germany)

### Synthesis of phosphonate-functionalized porphyrins

The phosphonate-functionalized porphyrins were synthesized as metal-free variants employing our recent research methodologies. The synthesis of 5,10,15,20-tetrakis[p-(diisopropoxyphosphoryl)phenyl] porphyrin **(*p*-H₈TPPA-iPr₈)** and 5,10,15,20-Tetrakis [*p-*phenylphosphonic acid] porphyrin **(*p*-H₈TPPA)** is described in Maares et al. (2019)⁴⁹. 5,10,15,20-Tetrakis [*m*-phenylphosphonic acid] porphyrin **(*m*-H₈TPPA)** was synthesized in a Pd-catalyzed Arbuzov reaction according to Yücesan et al. (2020)⁵⁰. All compounds were separately dissolved as 10 mM stock solutions in DMSO.

### General measurements of absorption and fluorescence spectroscopy

Absorption spectra were recorded with a Tecan Infinite M200 Reader (Tecan Austria GmbH, Grödig/Salzburg, Switzerland) from 350 to 500 nm. The same device was used in fluorescence mode to measure fluorescence excitation (350-500 nM; λ Em. 650 nm) and fluorescence emission (550-850 nm; λ Exc. 415 nm) spectra. 3D scanning was performed on a Spark^{®} multimode microplate reader (Tecan Austria GmbH, Grödig/Salzburg, Switzerland) from 350 to 450 nm (Exc.) and 555 to 850 nm (Em.) with a 5 nm step size, excitation and emission bandwidths of 5 nm, and a detector gain of 100. A typical reaction mix was made by combining equal volumes of 20 µM phosphonate porphyrins diluted in assay-buffer (50 mM HEPES in bidistilled water adjusted to pH 6.5 with sodium hydroxide solution; depleted of multivalent cations with Chelex^{®} 100 Resin pretreatment⁵¹) and a double-concentrated metal cation solution. Following 30 min incubations at 37°C absorption or fluorescence measurements were done. For redox conversion tests the CuSO₄ solutions were pretreated with 500 µM of L-ascorbic acid before admixing into the phosphonate porphyrin dilutions. To evaluate the effect of metal chelators, the metal cation-treated incubation mixtures were posttreated with 50 equivalents of EDTA or EGTA for 15 min before fluorescence emission readout.

### Time-correlated single photon counting (TCSPC)

Fluorescence decay curves were recorded on samples of *p*-H₈TPPA and *m*-H₈TPPA at a final concentration of 10 µM in assay-buffer solution in standard 1x1 cm glass cuvettes while admixing amounts of 100 µM and 1 mM CuSO₄ to achieve the actual cupric concentration. After addition the sample was gently mixed and incubated for 1 minute. Measurements were performed employing a Hamamatsu R5900 16-channel multi-anode photomultiplier tube (PMT) with 16 separate output (anode) elements and a common cathode and dynode system (PML-16C, Becker&Hickl, Berlin, Germany) as described in Schmitt et al. (2019)⁵². A 405 nm pulsed laser diode (LDH-405, Picoquant, Berlin) delivering 80 ps FWHM pulses, driven at a repetition rate of 20 MHz, was used for excitation. The fluorescence was observed via a 430 nm longpass filter (FF01-430/LP-25, AHF Analysentechnik, Tübingen, Germany). The decay curves were fitted employing a Levenberg-Marquardt algorithm for the minimization of the χ2 with Origin^{®} (Origin Inc. Illinois, USA)⁵².

### Cell culture

The human intestinal cell line Caco-2 (European Collection of Cell Cultures, Porton Down, UK) were routinely cultured at 37°C, 5% CO₂ and humidified atmosphere in Dulbecco's Modified Eagles Medium (DMEM), containing 10% fetal calf serum (FCS) 100 U/mL penicillin and 100 µg/ml streptomycin. For cellular phosphonate porphyrins experiments, Caco-2 cells were transferred into 96 wells (initially seeding 5000 cells per well) and cultured for 14 days for differentiation into an enterocyte-like monolayer^{53, 54}. For phosphonate labeling, differentiated cells were treated with either *m*-H₈TPPA, *p*-H₈TPPA or *p*-H₈TPPA-iPr₈ in a HEPES-based incubation buffer (10 mM HEPES, pH 7.35, 120 mM NaCl, 5.4 mM KCI, 5 mM glucose, 1.3 mM CaCl₂, 1 mM MgCl₂, 1 mM NaH₂PO₄, 0.3 % bovine serum albumin) for 30 min. Excess fluorescent dye was removed by multiple washing steps before fluorescence emission scanning using 416 nm excitation (Tecan Infinite M200 reader; Tecan, Grödig/Salzburg, Germany). To analyze metal responsiveness of the intracellular phosphonate porphyrins, cells were post-treated with 50 µM of different metal solutions at 37°C in an albumin-free incubation buffer in a time-course experiment.

### Confocal imaging

Fluorescence images of phosphonate porphyrin-labeled Caco-2 enterocytes were acquired on a Leica TCS SP8 laser scanning confocal microscope equipped with LAS X 3.5.5.19976 software platform, using a HC PL APO CS2 63x/1.20 water objective. The filters settings were λ_{Exc} 488 nm/ λ_{Em} 580 nm; pinhole 111.5 µm, pinhole size 1 AU.

### References

1. Waldron, K. J., Rutherford, J. C., Ford, D. & Robinson, N. J. Metalloproteins and metal sensing. Nature 460, 823-830 (2009).
2. Andreini, C., Bertini, I., Cavallaro, G., Holliday, G. L. & Thornton, J. M. Metal ions in biological catalysis: From enzyme databases to general principles. J. Biol. Inorg. Chem. 13, 1205-1218 (2008).
3. Martínez-Fábregas, J., Rubio, S., Diaz-Quintana, A., Diaz-Moreno, I. & De la Rosa, M. Á. Proteomic tools for the analysis of transient interactions between metalloproteins. FEBS J. 278, 1401-1410 (2011).
4. Kambe, T., Tsuji, T., Hashimoto, A. & Itsumura, N. The physiological, biochemical, and molecular roles of zinc transporters in zinc homeostasis and metabolism. Physiol. Rev. 95, 749-784 (2015).
5. Haase, H. & Rink, L. Zinc signals and immune function. BioFactors 40, 27-40 (2014).
6. Grubman, A. & White, A. R. Copper as a key regulator of cell signalling pathways. Expert Rev. Mol. Med. 16, e11 (2014).
7. Wolfgang Maret, A. W. Binding, Transport and Storage of Metal Ions in Biological Cells. (Royal Society of Chemistry, 2014). doi:10.1039/9781849739979
8. Collins, J. F. Molecular, Genetic, and Nutritional Aspects of Major and Trace Minerals. Mol. Genet. Nutr. Asp. Major Trace Miner. (Elsevier Inc., 2016). doi:10.1016/c2014-0-02224-1
9. Kardos, J., Héja, L., Simon, A., Jablonkai, I., Kovács, R. & Jemnitz, K. Copper signalling: causes and consequences. Cell Commun Signal 16, 1-22 (2018).
10. Bogdan, A. R., Miyazawa, M., Hashimoto, K. & Tsuji, Y. Regulators of Iron Homeostasis: New Players in Metabolism, Cell Death, and Disease HHS Public Access. Trends Biochem Sci 41, 274-286 (2016).
11. Nielsen, A. E., Bohr, A. & Penkowa, M. The Balance between Life and Death of Cells: Roles of Metallothioneins. Biomark. Insights 1, 99-111 (2006).
12. Yu, S. P., Canzoniero, L. M. T. & Choi, D. W. Ion homeostasis and apoptosis. Curr. Opin. Cell Biol. 13, 405-411 (2001).
13. Mordang, J. J., Gubern-Mérida, A., Bria, A., Tortorella, F., Mann, R. M., Broeders, M. J. M., den Heeten, G. J. & Karssemeijer, N. The importance of early detection of calcifications associated with breast cancer in screening. Breast Cancer Res. Treat. 167, 451-458 (2018).
14. Norouzi, S., Adulcikas, J., Sohal, S. S. & Myers, S. Zinc transporters and insulin resistance: Therapeutic implications for type 2 diabetes and metabolic disease. J. Biomed. Sci. 24, 87 (2017).
15. Lowe, J., Taveira-da-Silva, R. & Hilário-Souza, E. Dissecting copper homeostasis in diabetes mellitus. IUBMB Life 69, 255-262 (2017).
16. Khan, A. R. & Awan, F. R. Metals in the pathogenesis of type 2 diabetes. J. Diabetes Metab. Disord. 13, 1-6 (2014).
17. Voelkl, J., Tuffaha, R., Luong, T. T. D., Zickler, D., Masyout, J., Feger, M., Verheyen, N., Blaschke, F., Kuro-o, M., Tomaschitz, A., Pilz, S., Pasch, A., Eckardt, K. U., Scherberich, J. E., Lang, F., Pieske, B. & Alesutan, I. Zinc inhibits phosphate-induced vascular calcification through TNFAIP3-mediated suppression of NF-kB. J. Am. Soc. Nephrol. 29, 1636-1648 (2018).
18. Vallejo-Illarramendi, A., Toral-Ojeda, I., Aldanondo, G. & López de Munain, A. Dysregulation of calcium homeostasis in muscular dystrophies. Expert Rev. Mol. Med. 16, e16 (2014).
19. Bush, A. I., Pettingell, W. H., Multhaup, G., Paradis, M. D., Vonsattel, J. P., Gusella, J. F., Beyreuther, K., Masters, C. L. & Tanzi, R. E. Rapid induction of Alzheimer Aβ amyloid formation by zinc. Science (80-.). 265, 1464-1467 (1994).
20. Zatta, P., Drago, D., Bolognin, S. & Sensi, S. L. Alzheimer's disease, metal ions and metal homeostatic therapy. Trends Pharmacol. Sci. 30, 346-355 (2009).
21. Liu, Y., Nguyen, M., Robert, A. & Meunier, B. Metal Ions in Alzheimer's Disease: A Key Role or Not? Acc. Chem. Res. 52, 2026-2035 (2019).
22. Maret, W. Molecular aspects of human cellular zinc homeostasis: Redox control of zinc potentials and zinc signals. in BioMetals 22, 149-157 (2009).
23. Ackerman, C. M., Lee, S. & Chang, C. J. Analytical Methods for Imaging Metals in Biology: From Transition Metal Metabolism to Transition Metal Signaling. Anal. Chem. 89, 22-41 (2017).
24. Carter, K. P., Young, A. M. & Palmer, A. E. Fluorescent Sensors for Measuring Metal Ions in Living Systems. Chem. Rev. 114, 4564-4601 (2014).
25. Hong, G., Antaris, A. L. & Dai, H. Near-infrared fluorophores for biomedical imaging. Nat. Biomed. Eng. 1, 1-22 (2017).
26. McRae, R., Bagchi, P., Sumalekshmy, S. & Fahrni, C. J. In situ imaging of metals in cells and tissues. Chem. Rev. 109, 4780-4827 (2009).
27. New, E. J., Wimmer, V. C. & Hare, D. J. Promises and Pitfalls of Metal Imaging in Biology. Cell Chem. Biol. 25, 7-18 (2018).
28. Nolan, E. M. & Lippard, S. J. Small-molecule fluorescent sensors for investigating zinc metalloneurochemistry. Acc. Chem. Res. 42, 193-203 (2009).
29. Frederickson, C. J. Neurobiology of Zinc and Zinc-Containing Neurons. Int. Rev. Neurobiol. 31, 145-238 (1989).
30. Burdette, S. C., Walkup, G. K., Spingler, B., Tsien, R. Y. & Lippard, S. J. Fluorescent sensors for Zn2+ based on a fluorescein platform: Synthesis, properties and intracellular distribution. J. Am. Chem. Soc. 123, 7831-7841 (2001).
31. Zhang, X. A., Hayes, D., Smith, S. J., Friedle, S. & Lippard, S. J. New strategy for quantifying biological zinc by a modified zinpyr fluorescence sensor. J. Am. Chem. Soc. 130, 15788-15789 (2008).
32. Hurst, T. K., Wang, D., Thompson, R. B. & Fierke, C. A. Carbonic Anhydrase II-Based Metal Ion Sensing: Advances and New Perspectives. Biochim Biophys Acta 1804, 393-403 (2010).
33. Bischof, H., Burgstaller, S., Waldeck-Weiermair, M., Rauter, T., Schinagl, M., Ramadani-Muja, J., Graier, W. F. & Malli, R. Live-Cell Imaging of Physiologically Relevant Metal Ions Using Genetically Encoded FRET-Based Probes. Cells 8, 492 (2019).
34. Aper, S. J. A., Dierickx, P. & Merkx, M. Dual Readout BRET/FRET Sensors for Measuring Intracellular Zinc. ACS Chem. Biol. 11, 2854-2864 (2016).
35. Vinkenborg, J. L., Nicolson, T. J., Bellomo, E. A., Koay, M. S., Rutter, G. A. & Merkx, M. Genetically encoded FRET sensors to monitor intracellular Zn2+ homeostasis. Nat. Methods 6, 737-740 (2009).
36. Schmitt, F. J., Thaa, B., Junghans, C., Vitali, M., Veit, M. & Friedrich, T. EGFP-pHsens as a highly sensitive fluorophore for cellular pH determination by fluorescence lifetime imaging microscopy (FLIM). Biochim. Biophys. Acta - Bioenerg. 1837, 1581-1593 (2014).
37. Schmitt, F. J., Maksimov, E. G., Suedmeyer, H., Jeyasangar, V., Theiss, C., Paschenko, V. Z., Eichler, H. J. & Renger, G. Time resolved temperature switchable excitation energy transfer processes between CdSe/ZnS nanocrystals and phycobiliprotein antenna from Acaryochloris marina. in Photonics Nanostructures - Fundam. Appl. 9, 190-195 (2011).
38. Velazquez Escobar, F., Hildebrandt, T., Utesch, T., Schmitt, F. J., Seuffert, I., Michael, N., Schulz, C., Mroginski, M. A., Friedrich, T. & Hildebrandt, P. Structural parameters controlling the fluorescence properties of phytochromes. Biochemistry 53, 20-29 (2014).
39. Vitali, M., Bronzi, D., Krmpot, A. J., Nikolić, S. N., Schmitt, F. J., Junghans, C., Tisa, S., Friedrich, T., Vukojević, V., Terenius, L., Zappa, F. & Rigler, R. A Single-Photon Avalanche Camera for Fluorescence Lifetime Imaging Microscopy and Correlation Spectroscopy. IEEE J. Sel. Top. Quantum Electron. 20, 344-353 (2014).
40. Märk, J., Schmitt, F.-J., Theiss, C., Dortay, H., Friedrich, T. & Laufer, J. Photoacoustic imaging of fluorophores using pump-probe excitation. Biomed. Opt. Express 6, 2522-2535 (2015).
41. Märk, J., Schmitt, F.-J. & Laufer, J. Photoacoustic imaging of the excited state lifetime of fluorophores. J. Opt. 18, 054009 (2016).
42. Tejwani, V., Schmitt, F. J., Wilkening, S., Zebger, I., Horch, M., Lenz, O. & Friedrich, T. Investigation of the NADH/NAD+ ratio in Ralstonia eutropha using the fluorescence reporter protein Peredox. Biochim. Biophys. Acta - Bioenerg. 1858, 86-94 (2017).
43. Linder, M. C. Ceruloplasmin and other copper binding components of blood plasma and their functions: an update. Metallomics 8, 887-905 (2016).
44. Rossi, A., Poverini, R., Di Lullo, G., Modesti, A., Modica, A. & Scarino, M. L. Heavy metal toxicity following apical and basolateral exposure in the human intestinal cell line Caco-2. Toxicol. Vitr. 10, 27-31 (1996).
45. Bannon, D. I., Abounader, R., Lees, P. S. J. & Bressler, J. P. Effect of DMT1 knockdown on iron, cadmium, and lead uptake in Caco-2 cells. Am J Physiol Cell Physiol 284, 44-50 (2003).
46. Vázquez, M., Calatayud, M., Vélez, D. & Devesa, V. Intestinal transport of methylmercury and inorganic mercury in various models of Caco-2 and HT29-MTX cells. Toxicology 311, 147-153 (2013).
47. Rae, T. D., Schmidt, P. J., Pufahl, R. A., Culotta, V. C. & O'Halloran, T. V. Undetectable intracellular free copper: The requirement of a copper chaperone for superoxide dismutase. Science (80-.). 284, 805-808 (1999).
48. Maares, M., Keil, C., Koza, J., Straubing, S., Schwerdtle, T., Haase, H., Maares, M., Keil, C., Koza, J., Straubing, S., Schwerdtle, T. & Haase, H. In Vitro Studies on Zinc Binding and Buffering by Intestinal Mucins. Int. J. Mol. Sci. 19, 2662 (2018).
49. Maares, M., Ayhan, M. M., Yu, K. B., Yazaydin, A. O., Harmandar, K., Haase, H., Beckmann, J., Zorlu, Y. & Yücesan, G. Alkali Phosphonate Metal-Organic Frameworks. Chem. - A Eur. J. 25, 11214 -11217 (2019).
50. Yücesan, G., Zorlu, Y., Brown, C., Keil, C., Ayhan, M. M., Haase, H., Thompson, R. B. & Lengyel, I. Fluorescent arylphosphonic acids: synergic interactions between bone and fluorescent core. Chem. - A Eur. J. chem.202001613 (2020). doi:10.1002/chem.202001613
51. Alker, W., Schwerdtle, T., Schomburg, L. & Haase, H. A zinpyr-1-based fluorimetric microassay for free zinc in human serum. Int. J. Mol. Sci. 20, 4006 (2019).
52. Schmitt, F.-J., Züleyha, ·, Campbell, Y., Mai, ·, Bui, V., Hüls, A., Tomo, T., Chen, · Min, Maksimov, E. G., Suleyman, ·, Allakhverdiev, I. & Friedrich, T. Photosynthesis supported by a chlorophyll f-dependent, entropy-driven uphill energy transfer in Halomicronema hongdechloris cells adapted to far-red light. Photosynth. Res. 139, 185-201 (2019).
53. Pinto, M., Robine-Leon, S., Appay, M.-D., Kedinger, M., Triadou, N., Dussaulx, E., Lacroix, B., Simon-assmann, P., Haffen, K., Fogh, J. & Zweibaum, A. Enterocyte-like Differentiation and Polarization. Biol. Cell 47, 323-330 (1983).
54. Ferraretto, A., Bottani, M., De Luca, P., Cornaghi, L., Arnaboldi, F., Maggioni, M., Fiorilli, A. & Donetti, E. Morphofunctional properties of a differentiated Caco2/HT-29 co-culture as an in vitro model of human intestinal epithelium. Biosci. Rep. 38, 1-16 (2018).

## Claims

1. A method for detecting free metal ions in a sample comprising,
- providing a liquid sample potentially comprising free metal ions,
- adding to said sample a fluorescent probe comprising
i. an organic fluorescent core and
ii. one or more metal binding functional group,
iii. wherein the one or more metal binding functional group is selected from the group comprising a phosphonic acid group and an arsonic acid group and is covalently linked to a sp or a sp²-carbon atom or a nitrogen atom of the fluorescent core via a P or As atom,
- and measuring fluorescence of said sample.

2. The method according to the preceding claim, wherein the organic fluorescent core is selected from the group comprising tetrapyrrole derivatives, such as porphyrin or phthalocyanine, acridine, BODIPY, cyanine or cyanine derivatives, carbazole, coumarin or coumarin derivatives, xanthene or xanthene derivatives such as fluorescein or rhodamine.

3. The method according to any one of the preceding claims, wherein the fluorescent probe comprises two or more metal binding functional groups.

4. The method according to any one of the preceding claims, wherein the fluorescent probe is selected from the group comprising
- 5,10,15,20-tetrakis[*p*-phenylphosphonic acid] porphyrin (*p*-H₈TPPA), and
- 5,10,15,20-tetrakis[*m*-phenylphosphonic acid] porphyrin (*m*-H₈TPPA).

5. The method according to any one of the preceding claims, wherein measuring fluorescence is performed by exciting the sample with a light of an excitation wavelength and detecting emitted light at an emission wavelength.

6. The method according to any one of the preceding claims, wherein the metal ions are ions of a group of metals comprising Cu, Zn, Pb, Hg, Cd, Co and Mn, wherein the ions are preferably divalent.

7. The method according to the preceding claim, wherein presence of the metal ions leads to a concentration-dependent decrease, increase or shift of fluorescence compared to a reference sample, wherein the method preferably involves determining a concentration of metal ions in said sample.

8. The method according to any one of the preceding claims, wherein the metal ions are Cu ions, preferably Cu²⁺ ions, and the fluorescent probe is *p*-H₈TPPA or *m*-H₈TPPA.

9. The method according to any one of claims 1-5, wherein the metal ions are ions of a group of metals comprising Fe, Mg and Ca, wherein the ions are preferably divalent.

10. The method according to the preceding claim, wherein presence of the metal ions leads to a concentration dependent increase of fluorescence compared to a reference sample, wherein the method preferably involves determining a concentration of metal ions in said sample.

11. The method according to any one of the preceding claims, wherein the liquid sample is a biological sample, such as a bodily fluid, a tissue sample or a sample comprising cells.

12. The method according to any one of the preceding claims, wherein the sample comprises cells and the fluorescent probe is cell permeable.

13. The method according to the preceding claim, wherein the fluorescence measurement is performed in association with a microscopic analysis of the cells, such as confocal fluorescent microscopy.

14. The method according to any one of the preceding claims, where the concentration of free metals is calculated based on the mass action law, in particular involving calibration based on addition of a chelator and/or metal ions

15. The method according to any one of the preceding claims, wherein the method is used in clinical diagnostics for determining available levels of free metal ions in a patient sample.
